Europäisches Patentamt

European Patent Office

Office européen des brevets

(11) Veröffentlichungsnummer: **0 222 366 B1**

(12) **EUROPÄISCHE PATENTSCHRIFT**

(45) Veröffentlichungstag der Patentschrift: **15.01.92**

(21) Anmeldenummer: **86115621.4**

(22) Anmeldetag: **11.11.86**

(51) Int. Cl.⁵: **C12N 15/56**, C12N 15/81, C12N 1/16, C12N 1/20, C12N 5/00, C12N 9/36, A61K 37/54

(54) **Verfahren zur Herstellung von Humanlysozym.**

(30) Priorität: **12.11.85 DE 3540075**

(43) Veröffentlichungstag der Anmeldung:
**20.05.87 Patentblatt 87/21**

(45) Bekanntmachung des Hinweises auf die Patenterteilung:
**15.01.92 Patentblatt 92/03**

(84) Benannte Vertragsstaaten:
**AT BE CH DE ES FR GB GR IT LI LU NL SE**

(56) Entgegenhaltungen:
EP-A- 0 123 544          EP-A- 0 129 073
EP-A- 0 155 189          EP-A- 0 181 634
EP-A- 0 208 472          BE-A- 901 223

Chemical abstracts, band 100, N 1, 2 January 1984, P. 132, Ref. N 1490e, Columbus, Ohio, US. H. Land et al "Synthesis of ds-cDNA involving addition of dCMP tails to allow cloning of 5'-terminal mRNA sequences" & Methods enzymol, 1983, 100 (Recomb. DNA, Pt B), 285-92

(73) Patentinhaber: **BOEHRINGER INGELHEIM INTERNATIONAL GmbH**
**Postfach 20**
**W-6507 Ingelheim am Rhein(DE)**

(72) Erfinder: **Sledziewski, Andrzej, Dr.**
**9010 Northeast 27th Avenue**
**Seattle 98115(US)**
Erfinder: **Chlebowicz-Sledziewska, Eva, Dr.**
**9010 Northeast 27th Avenue**
**Seattle 98115(US)**
Erfinder: **Swetly, Peter, Prof. Dr.**
**Hietzinger Hauptstrasse 40B/9**
**A-1130 Wien(AT)**
Erfinder: **Adolf, Günther, Dr.**
**Johannagasse 20/7**
**A-1050 Wien(AT)**
Erfinder: **Hauptmann, Rudolf, Dr.**
**Döllachgasse 22**
**A-2483 Ebreichsdorf(AT)**

Nucleic acids Research, Band 5, N 9, September 1978, p. 3275-3294, Inf. Retrieval Ltd, London, GB. A.E. Sippel et al "Cloning of chicken lysozyme structural gene sequences synthesized in vitro"

Gene, Band 25, 1983, Ref. N 876, p. 263-269 ; Elsevier Sc. Publ. U. Gubler et al "A simple and very efficient method for generating cDNA libraries"

Agricultural and Biological Chemistry, Band 49, n 9, September 1985, p. 2829-2831, Tokyo, JP. M. Muraki et al "Expression of synthetic human lysozyme gene in Escherichia coli"

Gene. Band 34, n 1, 1985, Ref. n 1190, p. 9-15, Elsevier Sc. Publ. Amsterdam, NL. H.A. Vadavada et al "Cloning and expression of genes for lysozymes and a 20-k Dal protein of colitis bacteriophage"

Erfinder: **Castanon, Maria Josefa, Dr.**
**Costenoblegasse 2-3-8**
**A-1113 Wien(AT)**
Erfinder: **Spevak, Walter, Dr.**
**Ernstbrunnenstrasse 2-1-4**
**A-2000 Stockerau(AT)**

**Beschreibung**

Gegenstand der vorliegenden Erfindung sind für Human-Lysozym-Protein codierende, mit einer Hefe-Leadersequenz ausgestattete DNA-Moleküle, damit rekombinierte DNA-Moleküle sowie das Verfahren zur Herstellung von aktivem Human-Lysozym.

Gegenstand der Erfindung sind außerdem Vektoren, insbesondere Expressions-Vektoren und -Plasmide, mit den für Human-Lysozym codierenden und mit einer Hefe-Leadersequenz ausgestatteten Nukleotid-Sequenz als Inserts, sowie verschiedene Wirtsorganismen oder Kulturen, die die Herstellung von aktivem Human-Lysozym ermöglichen.

Lysozyme sind definiert als 1,4-Beta-N-acetylmuramidasen, die die glykosidische Bindung zwischen dem C-1 der N-acetyl-muraminsäure (MurNAc) und dem C-4 von N-acetylaglucosamin (GlcNAc) im Peptidoglykan von Bakterien spalten (Phillips, D.C., Sci. Am. 215, 78-90, 1966). Alexander Fleming entdeckte 1922, daß sowohl verschiedene Gewebe und Secrete als auch Hühnereiweiß einige Gram-positive Bakterien lysieren können. Er bezeichnete den lytischen Faktor als Lysozym, d.h. ein Enzym, das in der Lage ist, Bakterien zu lysieren. Fleming zeigte, daß Lysozym in Gewebehomogenaten aus Knorpel und Magen, in Tränen, Speichel, Sputum, Nasensekreten, pathologischem Urin, Serum und in Leukocyten vorkommt, nicht aber im Urin Gesunder, in der Cerebrospinalflüssigkeit oder im Schweiß (Fleming, A., Proc. Roy. Soc. SerB 93, 306-307, 1922).

Die Wirkungsweise des Lysozyms als antibakterielles Agens scheint sowohl durch seine direkte bakteriolytische Wirkung, als auch über stimulatorische Effekte im Zusammenhang mit der Phagocytose der Makrophagen zum Tragen zu kommen (Biggar, W.D., Sturgess, J.M., Infect. Immunol. 16, 974-982, 1977; Thacore, M., Willet, H.P., Am. Rev. Resp. Dis. 93, 786-790, 1966).

Die bedeutende Rolle des Lysozyms als Vermittler bei der Mikrobenabwehr durch alveoläre Macrophagen der Ratte konnte gezeigt werden: Bei intakten Bakterien erfolgte keine Phagocytose, während lysozymgeschädigte Bakterien schnell phagocytiert werden (Biggar, W.D., Sturgess, J.M., Infect. Immunol. 16, 974-982, 1977). Ebenso konnte nachgewiesen werden, daß Lysozym direkt die phagocytotische Aktivität von polymorphonuklearen Leukozyten (Klockars, M.I., Roberts, P., Acta Haematol. 55, 289-292, 1976) und von Makrophagen (Thacore, M., Willet, H.P., Am. Rev. Resp. Dis. 93, 786-790, 1966) erhöhen kann. Untersuchungen zur Wirkungsweise von Lysozym auf Mikroorganismen des Mundes wurden an sieben Serotypen von Streptococcus mutans, Veillonella alcalescens und an virulenten und nicht-virulenten Stämmen von Actionomyces viscosus T 14 durchgeführt. Die Ergebnisse zeigten, daß unterschiedliche Mechanismen für die bakteriostatische, lytische und bakteriozidale Eigenschaften verantwortlich sein können und daß das Enzym nicht nur ein selektiver sondern auch ein effektiver Faktor gegen Mikroorganismen des Mundes darstellt (Iacono, V.J. et al., Infect. Immunol. 29, 623-632, 1980). Andere postulierte Funktionen des Lysozyms umfassen die Immunstimulation (Jolles, P., Biomedecine 25, 275-276, 1976), die immunologische und nicht immunologische Kontrolle von Wirts-Membranen hinsichtlich einer neoplastischen Transformation (Ossermann, E.F., Adv. Pathobiol. 4, 98-102, 1976). Die Lysozym-Bestimmung aus Serum und/oder Urin wird zur Diagnose verschiedener Krankheiten oder als Indikator für ihre Entwicklung benutzt. Bei der akuten lymphoblastischen Leukämie ist der Lysozym-Serumspiegel deutlich erniedrigt, während im Falle der chronischen myelotischen Leukämie und bei den akuten monoblastischen und myelomonocytischen Leukämien die Lysozymkonzentration im Serum stark erhöht ist (Jolles, P. et al., Isr. J. Med. Sci. 1, 445-447, 1965; Ossermann, E.F. and Lawlor, D.P., J. Exp. Med. 124, 921-952, 1966).

Die therapeutisch wirksame Anwendung von Lysozym ist denkbar bei der Behandlung verschiedener Bakterien- und Virus- (Zona, Herpes Zoster) Infektionen, bei Colitis, verschiedener Schmerzen, bei Allergien, Entündungen sowie in der Pädiatrie (Maternisierung von Kuhmilch durch Zugabe von Lysozym).

Lysozym vermag mit anderen biologisch aktiven Proteinen so zu interagieren, daß diese zur vollen Wirkungsentfaltung gelangen (Adinolfi, In: Lampert, Woods (eds), Academic Press, London, 1981, 19-47). Solche Komponenten wären z.B. Komplement, Laktotransferrin, das die Vermehrung bestimmter Mikroorganismen durch Bildung von Eisen-Chelat-Komplexen inhibiert, Antikörper wie sIgA der Milch,das die antibakterielle Wirkung des Laktotransferrins potenziert (Spik et al. Bull. Eur. Physiopath. Resp. 19, 123-130, 1983). Auch werden Lysozym, Laktotransferrin und Immunoglobuline koexistierend in verschiedenen natürlichen Sekreten wie Speichel, Tränenflüssigkeit, verschiedenen Milcharten (Jorieux et al. Protides of the biological Fluids, Proc. 31st Coll. 1984), in der Bronchialschleimhaut oder im Eiweiß vorgefunden. Als weiterer Einsatz ist Lysozym vorzugsweise einsetzbar zur Linderung rheumatischen Fiebers und rheumatischer Schmerzen und besitzt eine therapeutische Wirkung bei Krankheiten des rheumatischen-arthritischen Formenkreises (Third Int. Symp. on Flemings lysozyme, Milan 1964). Später wurde dem Lysozym auch analgetische Eigenschaften zugesagt (Bianchi, Eur. J. Pharmacol. 71, 211 - 221, 1981). In jüngerer Zeit konnte eine antinoziceptive Wirkungsweise von Lysozym gefunden werden (Bianchi, Clin. Exp. Pharmacol.

Physiol. 10, 45-52, 1983) Diese breite Palette der Wirkungsweise von Lysozym läßt ein entsprechend großes Spektrum für den therapeutischen Einsatz erkennen, so daß daraus ein bedeutender wirtschaftlicher Wert abzuleiten ist.

Bei allen hier aufgezählten pharmazeutischen Anwendungen von Lysozym sollte Human-Lysozym (HLZ) dem aus Hühnereiweiß gewonnenen Lysozym vorgezogen werden, da unerwünschte Nebenwirkungen anaphylaktischer und/oder allergischer Natur bei der Anwendung speziesfremder Lysozyme eher zu erwarten sind und einer Therapie entgegenstehen können.

Bis heute sind die Milch vom Menschen und die Plazenta des Menschen die einzigen kommerziellen Quellen zur Gewinnung von Human-Lysozym. Diese Ausgangsmaterialien sind jedoch sehr begrenzt erhältlich und es ist offensichtlich, daß von Ansatz zu Ansatz unterschiedliche Präparate erhalten werden. Durch die Nutzung der weitentwickelten industriellen Mikrobiologie und der jüngst entwickelten rekombinanten DNA-Technologie sollten sich Vorteile für die Herstellung von Human-Lysozym durch Mikroorganismen zeigen.

Das Gen für Hühnereiweiß-Lysozym konnte isoliert (Sippel, A.E. et al., Nucl. Acids Res. 5, 3275-3294, 1978; Baldacci, P. et al., Nucl. Acids Res. 6, 2667-2681, 1979) und die Nukleotid-Sequenz der Hühnereiweiß-Lysozym mRNA sowie die der auf dem Gen liegenden Exons zusammen mit ihren flankierenden Regionen bestimmt werden (Jung, A. et al., Proc. Natl. Acad. Sci. USA 77, 5759-5763, 1980). Ferner wurde die Nukleotid-Sequenz des Lysozym-Gens vom Bakteriophagen T4 aufgeklärt (Owen, J.E. et al., J. Mol. Biol. 165, 229-248, 1983).

Bekannt ist die Aminosäure-Sequenz von HLZ (Jolles, J. and Jolles, P., Helv. Chim. Acta 54, 2668-2675, 1971; Canfield, R.E., et al., Nature New. Biol. 232, 16-17, 1971; Jolles, P. and Jolles, J. Molec. and Cellul. Biochem. 63, 165-189, 1984), nicht aber die erfindungsgemäß dargestellte für HLZ codierende Nukeotid-Sequenz.

Die EP-A 181 634 beschreibt die Expression von Human-Lysozym in Hefe und Bacillus subtilis. Abgesehen davon, daß in der EP-A 181 634 kein spezieller Hefe-Terminator und ein synthetisches HLZ-Gen verwendet werden, besitzt diese HLZ-DNA-Sequenz im Falle der Expression in Hefe keine vorgeschaltete für ein Leaderpeptid codierende DNA-Sequenz. Das gebildete HLZ kann somit nicht aus dem Wirt transportiert werden, so daß die Ausbildung einer exakten Tertiärstruktur durch entsprechende Disulfidbrückenbildung erschwert wird. Es wird daher nicht gelehrt, wie man nach der EP-A 181 634 zu authentischem, löslichem und aktivem HLZ gelangt. Auch wird nicht gelehrt, wie das Startmethionin zu entfernen ist.

Die Publikation von M. Muraki, Agric. Biol. Chem. 49, 2829-2831, 1985, offenbart die Verwendung einer synthetischen, für Human-Lysozym kodierenden DNA-Sequenz und ihre Expression in E. coli. Dieses Verfahren führte zu einem unlöslichen und inaktiven Produkt, dessen Aktivität durch ein weiteres Regenerationsverfahren zurückgewonnen werden mußte, jedoch mit nur geringer Ausbeute an aktiven Human-Lysozym.

Die BE-A 901 223 beschreibt die Expression von Hühnerlysozym in Hefezellen unter Verwendung der bekannten Hühnerlysozym cDNA und einer der Prähühnerlysozym entsprechenden Signalsequenz. Von der hiernach erhaltenen Ausbeute an Hühnerlysozym (162 U/ml Kulturmedium) verbleibt jedoch ein erheblicher Anteil zellgebunden (44 U). Es wird ausgeführt, daß auch Lysozyme anderer Herkunft mit sehr ausgeprägter Homologie zur Hühnerlysozym DNA nach diesem Verfahren hergestellt werden können. Weiterführende Sequenzinformationen werden jedoch keine angegeben.

Die Aufgabe der vorliegenden Erfindung war es, die aufgeführten Nachteile zu beseitigen und über die Techniken der DNA-Rekombination ein lösliches, biologisch aktives Human-Lysozym bereitzustellen.

Die Aufgabe wurde erfindungsgemäß dadurch gelöst, daß ein DNA-Molekül bestehend aus a) einer Nukleotidsequenz der in Anspruch 1 angegebenen Formel und b) einer Hefe-Leadersequenz, welche mit dem 5'-Ende von a) verbunden ist, bereitgestellt wurde. Durch geeignete Rekombinationen mit entsprechenden vektoriellen Nukleotidsequenzen wird es ermöglicht, lösliches, aktives Human-Lysozym über geeignete transformierte Wirtszellen herzustellen.

Die HLZ-DNA, als Teil des Bakterien-Hybridvektors, kann von irgendwelchen HLZ-produzierenden Zellen des Menschen abstammen. Beispiel geeigneter Zellen sind Plazentazellen, Leukozyten von an akuter monoblastischer und/oder myelomonocytischer Leukämie erkrankter Menschen, Colonkarzinomzellen des Menschen, die U-937 Zellinien oder andere HLZ produzierende Zellinien. Als bevorzugter Gegenstand der vorliegenden Erfindung werden U-937 Zellen (ATCC CRL 1593) des Menschen benutzt. Die HLZ DNA kann aus einer Genbank, die das HLZ Gen enthält, isoliert werden. In der vorliegenden Erfindung wurde chromosomale DNA nicht bevorzugt, da sie wahrscheinlich innerhalb ihrer codierenden Region Introns enthält (z.B. enthält das Hühnereiweiß-Lysozym-Gen drei Introns (Jung, A. et al., 1980, loc. cit.)), die nicht durch Hefezellen herausgeschnitten werden können. In der vorliegenden Erfindung wurde Human-Lysozym-cDNA bevorzugt.

Die aus der Zelle U-937 isolierte mRNA ist ein bevorzugtes Template für die Synthese von Human-Lysozym cDNA. Für die Synthese des ersten Stranges der HLZ cDNA wird die Reaktion mit Oligo(dT)$_{18}$ als Primer gestartet, der vorzugsweise mit dem 3'Ende der besagten mRNA paart. In Gegenwart von dNTPs und der Reversen Transkriptase erfolgt die weitere Verlängerung der Einzelstrang cDNA. Die Einzelstrang cDNA kann anschließend in eine doppelsträngige cDNA durch verschiedene bekannte Methoden konvertiert werden. In der vorliegenden Erfindung wurde die Methode nach Gubler und Hoffmann (Grubler, U. and Hoffmann, B.J., Gene 25, 263-269, 1983) bevorzugt. Die Synthese des zweiten cDNA-Stranges aus der Einzelstrang cDNA erfolgte durch Behandlung des mRNA-Hybrids mit RNaseH und DNA Polymerase I in Gegenwart von dNTPs. Anhand dieser Methode wird eine doppelsträngige cDNA erhalten, die als solche direkt kloniert werden kann. Das Klonieren von Doppelstrang cDNA in Bakterien-Vektoren kann nach verschiedenen bekannten Methoden durchgeführt werden. Doppelsträngige cDNA kann direkt als "blunt end" Fragment, über einen synthetischen Linker oder über die als "Homopolymertailing" bekannte Methode kloniert werden. Im Falle des Klonierens von HLZ cDNA wurde die Homopolymertailing-Methode vorgezogen. Bei dieser Methode werden mit der terminalen Transferase Einzelstränge am 3'Ende der HLZ-DNA durch Addition von Nukleotiden (vorzugsweise dGTP) an ihren "blunt"- oder "staggered"-Enden gebildet. In einer separaten Reaktion wird das Bakterien-Plasmid mit einer Restriktionsendonuklease linearisiert (Im Falle des Klonierens von HLZ cDNA wurde der Vektor pUC9 bevorzugt) und mit komplementären Schwänzen (vorzugsweise dCTP) versehen, um an seinen 3'Enden komplementäre Einzelstränge zu erhalten. Im nächsten Schritt wird die homopolymer verlängerte Doppelstrang cDNA mit dem entsprechend komplementär geschwänzten Vektor gemischt, um diese unter geeigneten Bedingungen zu verknüpfen. Nach erfolgter Rekombination werden die CaCl$_2$-behandelten E. coli Zellen mit diesem Gemisch transformiert und auf selektiven Platten ausplattiert. Die HLZ cDNA enthaltenden Klone können nach verschiedenen bekannten Methoden identifiziert werden. In der vorliegenden Erfindung wurde das Screening mit radioaktiv markierten, synthetischen Oligonuleotiden bevorzugt. Anhand publizierter Aminosäure-Sequenz von HLZ Protein wurden zwei Sätze von Oligonukleotiden in der Länge von je 17 Basen synthetisiert. Aufgrund der Tatsache, daß verschiedene Codons ein und dieselbe Aminosäure spezifizieren können, enthielt jeder Satz ein Gemisch verschiedener Oligonukleotide.

Die Synthese aller möglichen Kombinationen sichert, daß einer der vorhandenen Oligonukleotide optimal mit dem HLZ Gen paart. Der Einsatz zweier unabhängiger Pools von 17-mer Oligonukleotiden verminderte die Möglichkeit, daß die "falschen" Positiven ausgewählt werden.

In den Screening-Experimenten werden die DNA jener Bakterienklone, die die cDNA Inserts tragen, entsprechend der bei der "Kolonie-Hybridisierung" angewandten Methode auf Nitrozellulosefilter übertragen. Die Nitrozellulosefilter werden danach unter geeigneten Bedingungen mit den radioaktiv markierten Pools der Human-Lysozym spezifischen 17-meren hybridisiert. Nach der Identifizierung entsprechender Klone, wird die Plasmid DNA jedes Klons präpariert und die Insertion nach der Methode von Maxam und Gilbert (chemisches Verfahren) oder Sanger (Synthese mit Didesoxynukleotiden) sequenziert. Die Bestimmung der DNA-Sequenz von der cDNA-Insertion aus positiven Klonen ermöglicht es, daraus die Aminosäure-Sequenz des potentiellen Proteins herzuleiten. Der Vergleich dieser Sequenz mit der publizierten HLZ-Aminosäuresequenz gestattet es festzustellen, ob die klonierte cDNA tatsächlich die kodierende Region für das HLZ Protein enthält.

Im allgemeinen können Plasmid-Vektoren, die aus Species stammen, die kompatibel mit den Wirtszellen sind, in Verbindung mit diesen Wirten verwendet werden. Der Vektor trägt üblicherweise neben einer Replikationsstelle Erkennungssequenzen, die es ermöglichen, in transformierten Zellen phänotypisch zu selektionieren. Zum Beispiel wird E.coli üblicherweise mit pBR322 transformiert, ein Plasmid das aus E. coli Spezies stammt (Bolivar, et al., Gene 2, 95 (1977). pBR322 enthält Gene für Ampicillin- und Tetracyclin-Resistenz und liefert damit einfache Mittel, transformierte Zellen zu identifizieren. Das pBR322-Plasmid oder auch andere müssen außerdem von sich aus Promotoren enthalten oder sie müssen dahingehend modifiziert sein, daß sie vom Mikroorganismus zur Expression ihrer eigenen Proteine verwendet werden können. Die Promotoren, die am häufigsten bei der Herstellung rekombinanter DNA verwendet werden, beinhalten die Beta-Lactamase-(Penicillinase) und Lactose-Promotor-Systeme (Chang et al., Nature 275, 615 (1978); Itakura et al., Science 198, 1056 (1977); Goeddel et al., Nature 281, 544 (1979)), und Tryptophan (trp) Promotor-Systeme (Goeddel et al., Nucleic Acids Res. 8, 4057 (1980); Europa-Anmeldung, Offenlegungs-Nr. 0036 776). Während die erwähnten die gebräuchlichsten Promotoren sind, sind darüberhinaus auch andere mikrobielle Promotoren entwickelt und benutzt worden. Die Gen-Sequenz für HLZ kann beispielsweise unter der Kontrolle des Leftward-Promotors des Bakteriophagen Lambda (P$_L$) eingesetzt werden. Dieser Promotor ist einer der als besonders stark bekannten Promotoren, der steuerbar ist. Die Steuerung wird möglich durch den Lambda-Repressor, von dem benachbarte Restriktionsschnittstellen bekannt sind.

Eine Mutation dieses Gens, das für einen thermolabilen Repressor codiert, kann in einem Vektor, der eine vollständige HLZ-Sequenz enthält, eingefügt werden. Wird die Temperatur auf 42° C erhöht, wird der Repressor inaktiviert und der Promotor bis zu seiner maximalen Konzentration exprimiert. Die Summe der mRNA, die unter diesen Bedingungen produziert wird, sollte ausreichend sein, um eine Zelle zu erhalten, die unter ihren neuen synthetischen Ribonukleinsäuren ungefähr 10% enthält, die von dem $P_L$-Promotor stammt.

Auf diese Weise ist es möglich, eine Klon-Bank zu etablieren, in der eine funktionelle HLZ-Sequenz in Nachbarschaft zu einer Ribosomen-Bindungsstelle plaziert wird in variierenden Abständen zu dem Lambda-$P_L$-Promotor. Diese Klone können dann überprüft und der mit der höchsten Ausbeute selektiert werden.

Die Expression und Translation einer HLZ-Sequenz kann auch unter Kontrolle anderer Regulationssysteme, die als "homolog" zu dem Organismus in seiner untransformierten Form gelten können, ablaufen. So enthält z.B. chromosomale DNA von einem Lactose-abhängigen E.coli ein Lactose oder Lac-Operon, der durch Ausschüttung des Enzyms Beta-Galactosidase den Lactose-Abbau ermöglicht.

Die Lac-Kontrollelemente können aus dem Bacteriophagen Lambda-plac5, der infektös für E.coli ist, erhalten werden. Das Lac-Operon des Phagen kann durch Transduktion aus derselben Bakterien-Spezies stammen. Regulationssysteme, die bei dem erfindungsgemäßen Verfahren Verwendung finden können, können aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Arabinose-Operator, Colicine $E_1$-Operator, Galactose-Operator, alkalische Phosphatase-Operator, trp-Operator, Xylose A operator, tac-Promotor u.ä. Vorzugsweise kann ein Hefe-Promotor auf die Weise an die HLZ kodierende Region gekoppelt werden, daß eine effektive Expression von HLZ gewährleistet ist. Speziell sollte der Hefe-Promotor direkt vor der für matures HLZ kodierenden Region positioniert werden - mit einem an dieser Verbindungsstelle insertiertem Translationsstartsignal (ATG). Weiterhin kann dieses HLZ Expressionsmodul in unterschiedliche Hefe-Vektoren insertiert werden, um diese dann, über bekannte Verfahren der Hefe-Transformation, in Hefe zu etablieren. Hefezellen, die Hybridplasmide enthalten, können in verschiedenen Medien kultiviert werden und das gewünschte Produkt HLZ kann daraus isoliert und nach bekannten Methoden gereinigt werden. Alternativ kann eine Hefe-Signal-Sequenz bei der Konstruktion des HLZ-Expressionsmoduls eingeführt werden. In diesem Fall sind Hefezellen, die mit solch einem Plasmid transformiert wurden, in der Lage, HLZ durch ihre Zellwand in das Kulturmedium auszuscheiden, aus dem das gewünschte Produkt -HLZ- gewonnen und weiter gereinigt werden kann.

Insbesondere sind Hefe-Zellen als Wirtsorganismus für die Expression bevorzugt, da Hefezellen leicht zu kultivieren sind, die Bedingungen zur Fermentation im großen Maßstab etabliert sind und Hefen frei von Endotoxinen sind. Daher können bei der vorliegenden Erfindung vorzugsweise eukaryotische Mikroorganismen, wie Hefekulturen, verwendet werden. Saccharomyces cerevisiae ist die am meisten verwendete unter den eukaryotischen Mikroorganismen, obwohl eine Anzahl anderer Spezies allgemein erhältlich ist.

Zur Expression in Saccharomyces wird beispielsweise das Plasmid YRp7 (Stinchcomb, D.T. et al. Nature 282, 39 (1979); Kingsman et al., Gene 7, 141 (1979); Tschumper, et al. Gene 10, 157 (1980)) und das Plasmid YEp 13 (Broach et al., Gene 8, 121-133 (1979)) üblicherweise verwendet. Das Plasmid YRp7 enthält das TRPI-Gen, das eine Selektionierungsmarkierung für eine Hefemutante, die unfähig ist, in tryptophan-freiem Medium zu wachsen, bereitstellt; beispielsweise ATCC Nr. 44076. Die TRPI-Deletion als Charakteristikum des Hefe-Wirt Genoms stellt dann ein wirksames Hilfsmittel dar, um die Transformation nachzuweisen, indem ohne Tryptophan kultiviert wird. Ganz ähnlich verhält es sich bei dem Plasmid YEp13, das das Hefe-Gen LEU 2, das zur Ergänzung einer LEU-2-Mutante verwendet werden kann, enthält. Geeignete Promotor Sequenzen für Hefe-Vektoren beinhalten die 5'-flankierende Region der ADHI (Ammerer, G., Methods in Enzymology 101, 192-201, 1983), 3-Phosphoglycerat Kinase (Hitzeman, et al., J. Biol. Chem. 255, 2073, 1980) oder anderer glykolytischer Enzyme (Kawasaki und Fraenkel, BBRC 108, 1107-1112 (1982)), wie Enolase, Glycerinaldehyd-3-phosphat-Dehydrogenase, Hexokinase, Pyruvatdecarboxylase, Phosphofructokinase, Glucose-6-Phosphat Isomerase, Phosphoglucose Isomerase und Glucokinase. Bei der Konstruktion geeigneter Expression-Plasmide können die mit diesen Genen assoziierten Terminationssequenzen ebenfalls in den Expressions-Vektor am 3'Ende der zu exprimierenden Sequenz eingesetzt werden, um Polyadenylierung und Termination der mRNA vorzusehen.

Andere Promotoren, die zudem noch den Vorteil der durch Wachstumsbedingungen kontrollierten Transkription besitzen, sind die Promotor-Regionen der Alkohol-Dehydrogenase-2, des Isocytochrom C, der sauren Phosphatase, der abbauenden Enzyme, die mit dem Stickstoff-Metabolismus gekoppelt sind, die oben erwähnte Glycerinaldehyd-3-Phosphat Dehydrogenase und der Enzyme, die für die Metabolisierung von Maltose und Galaktose verantwortlich sind. Promotoren, die durch den Hefe Mating Typ Locus reguliert werden, beispielsweise Promotoren der Gene BARI, MEC1, STE2, STE3, STE5 können bei temperaturregulierten Systemen durch die Verwendung von temperaturabhängigen sir Mutationen eingesetzt werden. (Rhine, Ph.D. Thesis, University of Oregon, Eugene, Oregon (1979); Herskowitz and Oshima, The Molecular Biology of the Yeast Saccharomyces, part I, 181-209 (1981), Cold Spring Harbor Laboratory). Diese Mutationen beeinflussen die Expression der ruhenden Mating Typ Kassetten von Hefen und dadurch indirekt die Mating Typ abhängigen Promotoren. Generell ist jedoch jeder Plasmid Vektor, der einen Hefe-kompatiblen Promotor, originäre Replikations- und Terminationssequenzen enthält, geeignet.

Das Vorliegen der intakten HLZ cDNA-Sequenz, ermöglicht es, das HLZ-Gen für die Expression in anderen Mikroorganismen, wie z.B. Hefe, zu konstruieren. Für die erfindungsgemäße Expression von HLZ in Hefezellen können daher z.B. solche Sekretionsplasmide eingesetzt werden, die entweder aus den Elementen ADHI-Promotor - α-Faktor-Leader - HLZ-Gen - ADHII-Terminator oder aus den Elementen α-Faktor-Promotor - α-Faktor-Leader - HLZ-Gen - α-Faktor-Terminator zusammengesetzt sind. Die DNA-Sequenzen vom ADHI-Promotor, vom ADHII-Terminator und vom α-Faktor Gen sind bekannt (Jeffrey, L., et al., J. Biol. Chem. 257, 3018-25, 1982; Russell, D.W. et al., J. Biol. Chem. 258, 2674-82, 1983; Nucleic Acids Res. 12, 4049-63, 1983).

Ein auf diese Weise präpariertes HLZ-Gen kann funktionell mit einem ausgewählten Hefe-Promotor verbunden werden. Solch eine Expressionseinheit kann außerdem in verschiedenen Hefe-Vektoren integriert werden. Diese können Hefe transformieren, welches zur Synthese von Human-Lysozym führt.

Für die Expression von HLZ unter bevorzugter Kontrolle des ADHI-Promotors kann ein Plasmid konstruiert werden, das hinter dem 3'Ende des ADHI Promotors den kompletten α-Faktor-Leader enthält. An das 3'Ende des α-Faktor-Leaders wird die für matures HLZ kodierende DNA-Sequenz (HLZ-Gen) in der richtigen Orientierung zum α-Faktor-Leader ligiert. Bevorzugt wird eine Konstruktion, die einen exakten N-Terminus des gebildeten HLZ entstehen läßt. Für die endgültige Konstruktion zur Expressionskassette wird die ADHII-Terminatorsequenz, die die einheitliche Termination des HLZ-Gens bewirkt und zur Stabilität der mRNA beiträgt, so eingebaut, daß sie direkt im Anschluß an das Stop-Codon des HLZ-Gens zu liegen kommt. So ein Expressionsplasmid enthält alle beschriebenen Elemente in der richtigen Reihenfolge und Orientierung zueinander, sowie die exakten Übergänge zwischen den Elementen ADHI-Promotor, α-Faktor-Leader, HLZ-Gen und ADHII-Terminator. Besonders vorteilhaft ist es, wenn die Verbindung zwischen dem α-Faktor-Leader und dem darauffolgenden HLZ-Gen derart hergestellt wird, daß die für die Reifung der α-Faktors verantwortliche Proteaseschnittstelle nach LYS ARG exakt vor der Sequenz für die erste Aminosäure des reifen HLZ liegt, sodaß ein exakter N-Terminus des durch die Wirtszelle gebildeten HLZ-Proteins erhalten wird.

Eine derartige Expressionskassette kann in verschiedene Hefeexpressionsplasmide ligiert werden. Vorzugsweise kommen dafür die Multicolyplasmide YE$_p$13 (Broach, J.R. et al. Gene 8, 121-133, 1979), PJDB207 (DSM 3181, hinterlegt am 28.12.1984), Ylp5 (Struhl, K. et al., Proc. Natl. Acad. Sci. USA 76, 1035-1039, 1979) und pEAS102 in Frage. Der Vektor pEAS102 kann erhalten werden, indem Ylp5 mit PstI teilweise und mit BamHI vollständig verdaut und das isolierte 4.3 kb Fragment (enthält das URA3 Gen) mit dem 4.4kb BamHI/PstI-Fragment von pJDB207 ligiert werden.

Mit diesen, eine solche Expressionskassette tragenden Hefevektoren können Hefezellen beider mating-typen nach bekannten Verfahren transformiert werden. Das durch solche Transformanten produzierte und in das Kulturmedium freigesetzte HLZ kann nach bekannten Methoden der Proteinreinigung leicht isoliert werden.

Ebenso bevorzugt kann die Expression des HLZ-Gens auch unter der Kontrolle des α-Faktor-Promotors erfolgen. Hierzu wird die für matures HLZ codierende DNA-Sequenz in der richtigen Orientierung zwischen dem α-Faktor-Promotor und dem α-Faktor-Leader einligiert. Für die vollständige Konstruktion zur Expressionskassette werden α-Faktor-Promotor mit α-Faktor-Leader, HLZ-Gen und α-Faktor-Terminator hintereinander angeordnet. Auch in diesem Fall wird derselbe Übergang zwischen dem 3'Ende des α-Faktor-Leader und dem 5'-Ende des HLZ-Gens hergestellt, wie es bei der Herstellung der Expressionskassette unter der Kontrolle des ADHI-Promotors beschrieben worden ist. Eine solche Expressionskassette kann in die schon beschriebenen Hefeexpressionsplasmide eingebaut werden, mit denen dann nach bekannten Methoden Hefezellen transformiert werden können. Bevorzugte Wirte sind in diesem Fall Hefestämme des mating-type α. Die Kultivierung solcher Transformanten und die Isolierung des durch sie freigesetzten HLZ erfolgt nach bekannten Methoden.

Anhand dieser Konstruktionen ergeben sich eine Reihe von Vorteilen:

1. Einheitliche Terminierung der mRNA und somit größere Stabilität dieser mRNA

2. Einfache Isolierung von HLZ aus dem Überstand des Kulturmediums

3. Höherer Anteil an HLZ mit der richtigen Tertiärstruktur, da Disulfidbrücken im sezernierten Protein leichter gebildet werden können als innerhalb der Hefezelle.

4. Bei der Sekretion wird der Signal- oder Leaderpeptidanteil exakt vom maturen HLZ endoproteolytisch abgetrennt.

Dadurch erhält man einen genau definierten N-Terminus des Proteins. Das reife HLZ-Protein hat am N-Terminus ein Lysin. Die Plasmidkonstruktion kann nun so gewählt werden, daß beim Sekretionsplasmid vor dem Lysincodon die Proteaseschnittstelle positioniert wird. Dadurch ist die erste Aminosäure des sezernierten HLZ Lysin. Bei intrazellulärer Produktion muß vor die erste Aminosäure des reifen Lysozyms ein Start-ATG (Methionin) gesetzt werden, damit die Translation überhaupt beginnen kann. Dieses Methionin kann, wenn es durch einen zellulären Mechanismus nicht abgespalten wird, sehr störend sein (Aktivität, Stabilität, Antigenizität).

Zusätzlich zu Mikroorganismen sind Kulturen multizellulärer Organismen ebenfalls geeignete Wirte für die Expression von HLZ. Im Prinzip ist jede dieser Kulturen einsetzbar, ob von Wirbeltier- oder wirbellosen Tierzellkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßigen Methode wurde. (Tissue, Culture, Academic Press, Kruse and Patterson, Editors (1973). Beispiele solcher nützlichen Wirtszellinien sind VERO- und HeLa-Zellen, Goldhamster-Eierstock (CHO)-Zellen und WI38, BHK, COS-7 und MDCK-Zellinien.

Expressionsvektoren für diese Zellen enthalten üblicherweise (wenn nötig) eine Replikationsstelle, einen Promotor der vor dem zu exprimierenden Gen lokalisiert ist, gemeinsam mit jeder notwendigen Ribosomenbindungsstelle, RNA-Spleißstelle, Polyadenylierungsstelle und transkriptionelle Terminations-Sequenzen.

Bei der Verwendung in Säugetierzellen werden die Kontrollfunktionen auf den Expressions-Vektoren oftmals aus viralem Material vorgesehen. Beispielsweise stammen die üblicherweise verwendeten Promotoren aus Polyoma, Adenovirus 2, und besonders häufig aus Simian Virus 40 (SV 40). Die Anfangs- und Endpromotoren des SV 40 sind besonders nützlich, da beide leicht aus dem Virus als Fragment zu erhalten sind, das auch noch die virale Replikationsstelle des SV 40 enthält (Fiers et al., Nature 273, 113 (1978). Auch können kleinere oder größere Fragmente des SV 40 verwendet werden, vorausgesetzt, sie enthalten die annähernd 250 bp lange Sequenz die von der HindIII Schnittstelle bis zur BglI Schnittstelle in der viralen Replikationsstelle reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontrollsequenzen sind kompatibel zu den Wirtszellsystemen.

Eine Replikationsstelle kann entweder durch entsprechende Vektorkonstruktion vorgesehen werden, um eine exogene Stelle einzubauen, beispielsweise aus SV 40 oder anderen viralen Quellen (z.B. Polyoma, Adeno, VSV, PBV, etc.) oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Transformation der Zellen mit den Vehikeln kann durch eine Vielzahl an Verfahren erreicht werden. Beispielsweise kann sie durch Kalzium erfolgen, wobei entweder die Zellen in Magnesium gewaschen und die DNA den in Kalzium suspendierten Zellen zugegeben wird oder die Zellen einem Kopräzitipat von DNA und Kalziumphosphat ausgesetzt werden. Bei nachfolgender Genexpression werden die Zellen auf Medien übertragen, die für transformierte Zellen selektieren.Bei dem erfindungsgemäßen Polypeptid handelt es sich nicht ausschließlich nur um das reife Human-Lysozym (HLZ), das im einzelnen beschrieben ist, sondern ebenfalls um jedwede Modifikation dieses Polypeptides. Diese Modifikationen beinhalten z.B. Verkürzung des Moleküls am N- oder C-terminalen Ende, Austausch von Aminosäuren durch andere Reste, wodurch die Aktivität nicht wesentlich verändert wird.

Gegenstand der Erfindung sind nicht nur Gen-Sequenzen, die spezifisch für das angegebene HLZ codieren, sondern ebenfalls Modifikationen, die leicht und routinemäßig durch Mutation, Abbau, Transposition oder Addition erhalten werden können. Jede Sequenz, die für das beschriebene HLZ codiert (d.h. das das entsprechende und bekannte biologische Aktivitätsspektrum aufweist) und im Vergleich mit den gezeigten degeneriert ist, ist ebenfalls eingeschlossen; Fachleute auf diesem Gebiet sind in der Lage, DNA-Sequenzen der codierenden Regionen zu degenerieren. Ebenso ist jede Sequenz, die für ein Polypeptid mit dem Aktivitätsspektrum des HLZ codiert, und die mit den gezeigten Sequenzen (oder Teilen davon) unter stringenten Bedingungen hybridisiert (beispielsweise Bedingungen, die für mehr als 85%, bevorzugt mehr als 90% Homologie selektieren) beinhaltet.

Die erfindungsgemäße Substanz kann in bekannter Weise formuliert werden, um pharmazeutisch verwendbare Mittel zu erhalten, wobei das erfindungsgemäße Polypeptid entweder allein oder zusammen mit anderen Komponenten, wie verschiedenen Antibiotika (Tetrazyklin, Bacitracin), Enzymen (alpha-Amylase, Papain) oder Vitaminen, vermengt zur Verabreichung kommt.

Die folgenden Beispiele, die die Erfindung nicht eingrenzen sollen, beschreiben die Erfindung im Detail.

Kurze Beschreibung der Abbildungen

| Figur 1 | zeigt die Ergebnisse des Primer-Extensions-Experimentes, in dem Ei-ON19 und Ei-ON20 die Primer und das Template die aus der Zellinie U-937 isolierte RNA waren. |
|---|---|
| Figur 2 | zeigt die Ergebnisse der dot-blot-Hybridisierung. |
| Figur 3a | stellt die Struktur der HLZ cDNA (Klon HL 14-1) und |
| 3b | die Strategie für die Bestimmung der Nukleotidsequenz der HLZ cDNA dar. |
| Figur 4a | zeigt die DNA-Sequenz des HLZ cDNA Klons HL 14-1 |
| 4b | zeigt die Restriktionskarte der HLZ cDNA von Klon HL 14-1 |
| Figur 5 | zeigt den Vergleich zwischen publizierter HLZ-Aminosäuresequenz (obere Reihe) und der von der cDNA des Klons HL14-1 abgeleiteten Aminosäuresequenz (untere Reihe). |
| Figur 6 | zeigt den Multicopyhybridvektor pEAS 102 (Konstruktion und Verwendung siehe Text S. 15) |
| Figur 7 | zeigt die Verknüpfung des ADHI-Promotors mit dem $\alpha$-Faktor-Leader und die weitere Konstruktion zu pWS215D, in dem das HLZ-Gen in der richtigen Orientierung mit dem $\alpha$-Faktor-Leader verbunden ist. |
| Figur 8 | zeigt sowohl die Linkerligation mit HindIII-Linker (Beispiel 8b) als auch diejenige mit SalI- und HindIII-Linker (Beispiel 9b, führt zu pWS208D), zum Zweck des Einbaus des HLZ-Gens in der richtigen Orientierung zum $\alpha$-Faktor-Leader bzw. zum $\alpha$-Faktor-Promotor bzw. -Terminator. |
| Figur 9 | stellt die in vitro-Mutagenese mit den synthetischen Oligonukleotiden EBI 124 und EBI 234 dar mit dem Ziel, einen exakten Übergang zwischen dem $\alpha$-Faktor-Leader und dem HLZ-Gen herzustellen. |
| Figur 10 | zeigt die Konstruktion von pWS235D, in dem das exakte 3'-Ende des HLZ-Gens in der richtigen Orientierung vor dem ADHII-Terminator eingebaut wurde. |
| Figur 11 | zeigt oben die Entfernung der zwischen dem HLZ-Gen und dem ADHII-Terminator liegenden BamHI-Stelle (pWS235D) sowie die endgültige Konstruktion zur Expressionskassette (pWS290D). |
| Figur 12 | zeigt, wie das $\alpha$-Faktor-Gen aus dem pUC13-Derivat p$\alpha$F als EcoRI-Fragment in den Vektor V2 (puC18-Derivat, zur Konstruktion siehe Beispiel 9a) kloniert wurde (pWS230D). |
| Figur 13 | verdeutlicht den Weg, wie das HLZ-Gen in der richtigen Orientierung mit dem $\alpha$-Faktor-Promotor und -Terminator verbunden wurde (pWS231D) |
| Figuren 14a und 14b | zeigen die Entfernung der dem 5'-Ende des $\alpha$-Faktor-Gens am nächsten gelegenen PstI-Stelle (pWS294D) und die Fertigstellung der Expressionskassette pWS296D unter der Kontrolle des $\alpha$-Faktor-Promotors durch Ligierung des unter Abbildung 9 dargestellten mutagenisierten PstI-Fragments A2 in die einzige PstI-Stelle von pWS294D. |

## Experimenteller Teil

In den Beispielen benutzte Abkürzungen

| HLZ : | Human-Lysozym |
|---|---|
| RPMI : | Roswell Park Memorial Institut |
| TrisHCL : | Tris-(hydroxymethyl)-aminomethan, pH eingestellt mit HCl |
| EDTA : | Äthylendiamintetraessigsäure |
| DEP : | Diäthylpyrocarbonat |
| SDS : | Natriumdodecylsulfat |
| NaAc : | Natriumacetat |
| BSA : | Rinderserumalbumin |
| DTT : | 1,4-dithiothreitol (1,4-dimercapto-2,3-butandiol) |
| PCI : | Phenol: Chloroform: Isoamylalkohol (15:24:1) |
| Beta-NAD : | Beta-Nikotinamidadenindinukleotid |
| sscDNA : | Einzelstrang cDNA |
| E. coli : | Escherichia coli |
| SSC : | Lösung aus: 0.15M NaCl, 0,015M Natriumcitrat mit 10N NaOH auf pH 7 eingestellt. |
| Denhardt's Lösung: | Lösung aus 0,1 % Ficoll, 0,1 % Polyvinylpyrrolidon, 0,1 % BSA |

TBE : Lösung aus 0,82M Tris, 0.082M Borsäure, 0.002M EDTA pH8

Beispiel 1: HLZ-Aktivitätsbestimmung aus Zelline U-937

Es ist bekannt, daß die hämatopoietische Zellinie U-937 des Menschen Human-Lysozym produziert (Ralph, P. et al., J. Exp. Med. 143, 1528-1533, 1976). Um die Zellinie U-937 des Ausgangsmaterials der PolyA$^+$RNA für die Synthese von HLZ cDNA zu benutzen, wurden die Aktivität von HLZ bestimmt, das durch die U-937 Zellen in das Medium freigesetzt wurde. Eine frisch in RPMI 1640 Medium (10 % fetales Kälberserum, 100 Einheiten/ml (E/ml) Penicillin, 50 E/ml Streptomycin) inokulierte U-937 Zellinien-Kultur wurde bei 37°C für 3 Tage bis zur Dichte von 3 x 10$^5$ Zellen/ml kultiviert. Die Zellen wurden durch Zentrifugation entfernt und die Aktivität des ins Medium freigesetzten HLZ nach der Methode von Gold und Schweiger (Gold, L.M. and Schweiger, M., Methods in Enzymology 20, 537-542, 1971) bestimmt. Nach 2-stündiger Inkubation mit radioaktivem Substrat wurde die Menge an freigesetzter Radioaktivität in aliquoten Volumina von 100 μl, die aus Inkubationsmischungen von 2 ml entnommen wurden, gemessen. Als Negativkontrolle diente alleiniges RPMI-Medium. Die quantitative Bestimmung des von den U-937 Zellen freigesetzten HLZ erfolgte nach einer erstellten Standardkurve (Human-Lysozym-Aktivität aus Menschenmilch (Sigma) in RPMI Medium). In Tabelle 1 sind die Ergebnisse dargestellt.

Tabelle 1

| Verdünnungen des Mediums | CPM aus 100μl Inkubationsmischung | CPM aus 100 μl Medium | Menge an freigesetztem HLZ (μg/ml) |
|---|---|---|---|
| 2 x | 10 457 | 20 914 | 2.09 |
| 4 x | 3 963 | 15 852 | 1.59 |
| 10 x | 1 485 | 14 850 | 1.49 |
| 20 x | 794 | 15 880 | 1.59 |

Wie in Tabelle 1 dargestellt, setzen U-937 Zellen ungefähr 1,5 μg HLZ pro ml Medium in das RPMI-Medium frei. Daraus war zu schlußfolgern, daß die U-937 Zellinie eine brauchbare Quelle für die HLZ mRNA darstellt.

Beispiel 2: Präparation der Human-Lysozym mRNA enthaltenden RNA

a. Isolierung der Gesamt RNA

Die histiozytische Lymphom Zellinie U-937 vom Menschen wurde in RPMI Medium 1640 (10 % fetales Kälberserum, 100 E/ml Penicillin, 50 E/ml Streptomycin) bei 37°C kultiviert (Ralph, P. et al., 1976, loc. cit.). Zellen aus einer 1,6-Liter-Kultur wurden durch Zentrifugation gesammelt, einmal mit 50 ml eiskaltem 10 mM Tris HCl pH 7.4 / 140 mM NaCl / 1.5 mM MgCl$_2$ gewaschen und in 10 ml eiskaltem 10 mM Tris HCl pH 8.4 / 140 mM NaCl / 1.5 mM MgCl$_2$ resuspendiert. Das nicht-ionische Detergenz Nonidet P 40 wurde bis zur Endkonzentration von 0,5 % dazugegeben. Die Suspension wurde für 5 Minuten im Eis stehengelassen und danach für 10 Sekunden kräftig vermischt um die Zellmembranen zu lysieren. Die Nuclei wurden daraufhin durch Zentrifugation bei 4°C entfernt. Zu 10 ml Überstand wurden hinzugegeben: 0,5 Ml 20%iges SDS, 0,25 ml 2 M Tris HCl, P$_H$ 9.0, 0,1 ml 500 mM EDTA pH 7.5. Diese Probe wurde mit gleichem Volumen frisch destilliertem, mit 10mM Tris HCl pH 8 / 1 mM EDTA äquilibriertem Phenol versetzt und für 10 Minuten kräftig geschüttelt. Anschließend erfolgte die Phasentrennung durch Zentrifugation. Die wässrige Phase wurde zweimal wie oben beschrieben extrahiert und zum Schluß mit einem gleichem Volumen Chloroform. 3M Kaliumacetat (1/10 Volumen der wässrigen Phase) und anschließend 2,5 Volumen Äthanol wurden dazugegeben. Die Lösung wurde bei -20°C über Nacht stehengelassen, die präzipitierte RNA durch Zentrifugation gesammelt, einmal mit 3 ml 70%igem Äthanol gewaschen und im Vakuum getrocknet. Die RNA wurde in 3 ml Wasser gelöst, präzipitiert, gesammelt und getrocknet und wie oben wieder gelöst. Die Ausbeute betrug ungefähr 8 mg RNA.

b. Isolierung von polyA$^+$RNA

PolyA$^+$RNA wurde aus der Gesamt-RNA über oligo (dT)-Cellulose (P-L Biochemicals Inc.) isoliert. 0.5 g oligo (dT)-Cellulose wurde in sterilem Wasser suspendiert und bei 14°C über Nacht stehengelassen. Am nächsten Tag wurde eine mit DEP-behandeltem, sterilem Wasser gewaschene Säule (Biorad Plastic, 18 cm, Durchmesser 1,5 cm) mit 0,5 g oligo (dT)-Cellulose gepackt, mit 3 ml Proteinase K (Typ XI, Sigma) Lösung gewaschen und für 15 Minuten bei Raumtemperatur stehengelassen. Die Proteinase K war in 0.1 x Packungspuffer (1 x Packungspuffer: 50mM Tris HCl pH 7.4 1M NaCl, 1 mM ETDA, 0,2 % SDS) bis zur Endkonzentration von 1 mg/ml gelöst worden. Gewaschen wurde die Säule anschließend mit 5 ml steriler 0,1M NaOH, danach mit sterilem $H_2O$; bis das Eluat einen pH von 8 erreichte und schließlich mit 10 ml 1 x Packungspuffer. Die in 500 $\mu$l sterilem $H_2O$ resuspendierte Gesamt-RNA wurde mit 4.5 ml Packungspuffer verdünnt, für 5 Minuten bei 65°C erhitzt (Wasserbad) und damit die präparierte oligo (dT)-Cellulose Säule beladen. Das Eluat wurde gesammelt, nochmals bei 65°C für 5 Minuten erhitzt und wieder auf die Säule aufgetragen. Die Säule wurde mit 7 ml Packungspuffer gewaschen, um polyA RNA herauszueluieren. Fraktionen zu je 1 ml wurden gesammelt und die $OD_{260}$ jeder Fraktion gemessen. PolyA$^+$RNA wurde mit 5 ml sterilem Wasser eluiert. Sowohl polyA$^-$RNA als auch PolyA$^+$RNA wurden durch 0,3 M NaAc in Gegenwart von Äthanol bei -20°C über Nacht gefällt.

Beispiel 3: Test auf Vorhandensein von HLZ mRNA

a. Synthese von Oligonukleotiden mit gemischter Sequenz

Zwei Pools HLZ-homologer Oligonukleotide mit gemischter Sequenz wurden nach dem Festphasen-Phosphotriester-Verfahren (Alvarado-Urbina, G. et al., Science 214, 270-274, 1981) synthetisiert. Für die Konstruktion des 17-mer Oligonukleotid-Probengemisches Ei-ON19 wurde die Sequenz der Aminosäurereste 63-68 (Tyr Trp Cys Asn Asp Gly) vom $NH_2$-Terminus des Human-Lysozym Proteins (Jolles, J. and Jolles, P., 1971; Canfield, R.E. et al., 1971; Jolles, P. and Jolles, J. 1984; loc. cit.) herangezogen. Die Ei-ON19 Probe bestand aus einer Mischung von 16 Oligonukleotiden wie folgt:

$$5' \quad CC\frac{A}{G}TC\frac{A}{G}TT\frac{A}{G}CACCA\frac{A}{G}TA \quad 3'$$

Für die Konstruktion des 17-mer Oligonukleotid-Probengemisches Ei-ON20 wurde die Sequenz der Aminosäurereste 26-31 (Ala Asn Trp Met Cys Leu) vom $NH_2$-Terminus des Human-Lysozym Proteins (Jolles, J. and Jolles, P., 1971; Canfield, R.E. et al., 1971; Jolles, P. and Jolles, J. 1984; loc. cit.) herangezogen. Die Ei-ON20 Probe bestand aus einer Mischung von 32 Oligonukleotiden wie folgt:

$$5' \quad A\frac{AA}{GG}CACATCCA\frac{A}{G}TT\frac{\frac{A}{T}}{\frac{C}{G}}GC \quad 3'$$

b. Primer-Extension-Analyse

Die aus der U-937 Zellinie isolierte mRNA wurde nach der Primer-Extension-Methode auf das Vorhandensein von HLZ mRNA analysiert. Am 5'-Ende wurden 100 ng von Ei-ON19 oder von Ei-ON20 in einem Reaktionsansatz von 20 $\mu$l innerhalb von 60 Minuten bei 37°C radioaktiv markiert. Das Reaktionsgemisch bestand aus 70 mM Tris HCl pH 7.6, lmM $MgCl_2$, 30 $\mu$Ci (Gamma-$^{32}$p)ATP (5000 Ci/mmol, Amersham PB. 218) 100 $\mu$g/ml BSA, 10 mM DTT, lmM Spermidin und 2-4 Einheiten Polynukleotid Kinase (New England Biolabs). Die Reaktion wurde durch 10-minütiges Erhitzen des Gemisches bei 70°C beendet.

Die Primer-Extension-Reaktion ist in einem 40 $\mu$l-Reaktions-Volumen für 60 Minuten bei 42°C durchgeführt worden. Das Reaktionsgemisch bestand aus 50 mM Tris HCl, pH 8.3., 10 mM $MgCl_2$, 10 mM DDT, 4 mM Natriumpyrophosphat, 1.25 mM dGTP, 1.25 mM dATP, 1.25 mM dCTP, 1.25 mM dTTP, 150 ng aus der U-937 Zellinie isolierte mRNA, 40 ng radioaktivmarkierter Ei-ON19 oder Ei-ON20 und 200 Einheiten reverse Transkriptase (BRL). Die Reaktion wurde durch Zusatz von 50 $\mu$l 50 mM EDTA beendet, anschließend mit PCI extrahiert und mit Äthanol gefällt. Die Produkte der Primer-Extension wurden durch Zentrifugation gesammelt, getrocknet und in 20 $\mu$l einer angefärbten Formamidlösung, bestehend aus 90 % deionisiertem Formamid, 10 % 10 x TBE, 0,1 % Xylencyanol FF, 0.1 % Bromphenolblau, gelöst.

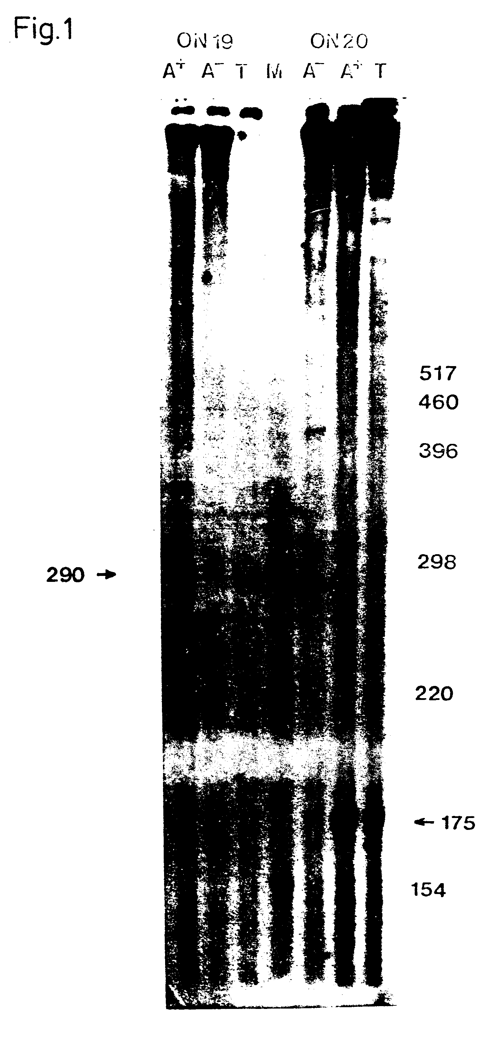

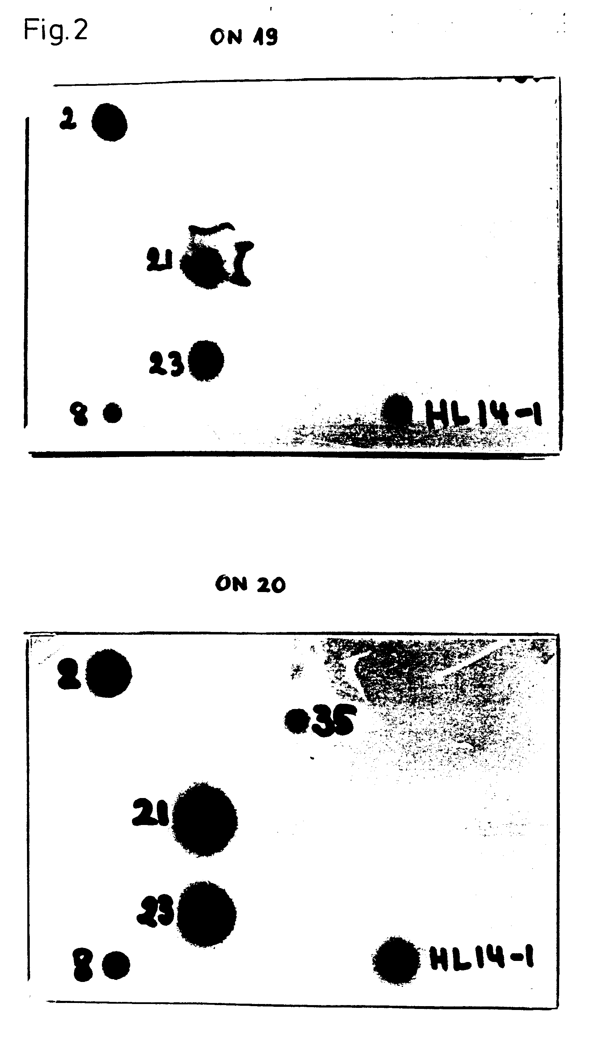

Von jeder Probe wurden 10 $\mu$l auf ein 5 %iges Polyacrylamid/8M Harnstoff-Gel aufgetragen. Die Elektrophorese erfolgte für 2 h bei 15 Watt. Nach Trocknen des Gels wurde damit ein Kodak X-Omat RP Röntgenfilm in einer Kodak X-Omatic Kassette C-2 geschwärzt. Die Ergebnisse dieses Experiments sind in Abbildung 1 dargestellt.

Da die beiden Primer Ei-ON19 und Ei-ON20 zu der HLZ mRNA komplementär sind, konnten nach der reversen Transkriptase-Reaktion bestimmte Extensionsprodukte von diesen auf dem HLZ mRNA-Template positionierten Primern erwartet werden (Lee, C.D. and Luse, D.C., Focus 4, 1-3, 1982). Da die Länge der HLZ mRNA unbekannt war, konnte die Länge des synthetisierten DNA nicht vorhergesagt werden. Es war jedoch folgendes festzustellen:

Der Primer Ei-ON19 paart an der Region auf der HLZ, die für die Aminosäurereste 63-68 codiert, sodaß die Länge der synthetisierten DNA mindestens 203 bp + X betragen muß (X ist die Anzahl derjenigen Nucleotide, die zur Leader-Sequenz und zur nicht-kodierenden 5'Region zählen). Die gleiche Berechnung ergibt für den Primer Ei-ON20 den Wert 92 bp + X. Es waren daher zwei Banden zu erwarten, die sich in ihrer Größe durch 111 bp unterscheiden sollten. Aus den Ergebnissen anhand Abbildung 1 ist zu ersehen, daß die mit dem Primer Ei-ON19 erhaltene stärkste Bande ungefähr 290 bp lang ist und die Länge der mit dem Primer Ei-ON20 erhaltenen stärksten Bande ungefähr 175 bp beträgt. Die Differenz zwischen diesen beiden Banden (115 bp) liegt sehr nahe bei dem vorherberechneten Wert und es war zu folgern, daß das analysierte mRNA-Präparat, welches aus der Zellinie U-937 isoliert wurde, HLZ mRNA enthält.

Beispiel 4: Konstruktion der U-937 cDNA Genbank

a. Synthese der cDNA

Die Synthese des ersten Stranges der cDNA wurde in einem Reaktionsvolumen von 50 $\mu$l mit folgender Zusammensetzung durchgeführt: 50 mM Tris HCl pH 8.3, 10 mM MgCl$_2$, 10 mM DTT, 4 mM Natriumpyrophosphat, 1.25 mM dGTP, 1.25 mM dATP, 1.25 mM dTTP, 0.5 mM dCTP, 20$\mu$Ci (Alpha-$^{32}$P) dCTP (3000 Ci / mmol. Amersham, PB. 10205), 100 $\mu$g/ml Oligo d(T)$_{18}$ (New England Biolabs), 40-100 $\mu$g/ml aus der Zellinie U-937 isolierte polyA$^+$RNA und 250 Einheiten reverse Transkriptase (BRL). Die Reaktion erfolgte während 60 Minuten bei 42$^°$C. Sie wurde durch Zugabe von 50 $\mu$l 50 mM EDTA beendet und die Reaktionsprodukte wurden mit PCI extrahiert. Das RNA/DNA-Hybrid wurde durch Äthanol/2M NH$_4$-Acetat bei -20$^°$C über Nacht präzipitiert. Die Mengen an synthetisierten ersten Strängen wurde durch Bestimmung TCA unlöslicher Radioaktivität ermittelt. Aus einer einzigen Reaktion wurden zwischen 200 und 300 ng einzelsträngige cDNA erhalten. Für die Synthese des zweiten Strangs wurde die Einzelstrang cDNA durch Zentrifugation pelletiert, einmal mit 80 %igem Äthanol gewaschen, getrocknet und in einem geeigneten Volumen H$_2$O dest. gelöst. Um die Einzelstrang-DNA (sscDNA) in doppelsträngige cDNA (dscDNA) zu konvertieren, können bis zu 500 ng sscDNA in einem Reaktionsvolumen von 100 $\mu$l verarbeitet werden. Das Reaktionsgemisch bestand aus 20 mM Tris HCl, pH 7.5, 5 mM MgCl$_2$, 10 mM (NH$_4$)$_2$ SO$_4$, 100 mM KCl, 0,15 mM Beta-NAD, 50 $\mu$g/ml BSA, 40 $\mu$M dNTPs, 8 E/ml E. coli RNaseH (BRL), 230 E/ml DNA Polymerase I (New England Biolabs), 10 E/ml E. coli DNA Ligase (New England Biolabs). Das Reaktionsgemisch wurde nacheinander für 60 Minuten bei 12$^°$C und für 60 Minuten bei 22$^°$C inkubiert. Die Reaktion wurde durch Zugabe von EDTA bis zur Endkonzentration von 20 mM beendet. Die dscDNA wurde mit PCI extrahiert und wie oben präzipitiert (Gubler, U. and Hoffmann, B., 1983, loc. cit.)

b. Klonierung der dscDNA Ein Klonierungsvektor mit niedrigem Transformationsbackground wurde wie folgt präpariert: 300 $\mu$g von pUC9 Plasmid-DNA wurden mit der Restriktionsendonuklease PstI vollständig verdaut. Die verdaute Plasmid-DNA wurde durch Agarosegel-Elektrophorese gereinigt, elektroeluiert und mit Äthanol präzipitiert. Ein Agarosegel-gereinigter Vektor erbringt in Transformationsexperimenten ungefähr 1-2 Kolonien / ng, während die Transformationsrate mit supercoiled pUC9 bei ungefähr 2000 - 4000 Kolonien pro ng Plasmid-DNA lag.

Mit der auf diese Weise präparierten pUC9 Plasmid-DNA erfolgte daraufhin in einem Reaktionsvolumen von 50 $\mu$l ein Homopolymertailing. Das Reaktionsgemisch bestand aus 100 mM Kalium-Cacodylat pH 7.5, 2mM CoCl$_2$, 0,2 mM DTT, 0 9 mM dCTP, 5 $\mu$Ci (5-$^3$H) dCTP (19 Ci/mmol, Amersham TRK. 352), 5 mg/ml BSA, 28 $\mu$g PstI - geschnittene pUC9 Plasmid-DNA und 45 Einheiten terminale Transferase (PL-Biochemicals). Das Reaktionsgemisch wurde für 5 Minuten bei 22$^°$C inkubiert und die Reaktion durch Zugabe von 50 $\mu$l 25 mM EDTA und anschließender Inkubation für 15 Minuten bei 70$^°$C beendet. Unter diesen Bedingungen wurden an jedem 3'-Ende 22 Nukeotide ansynthetisiert - eine Länge, die zur maximalen Transformationsrate benötigt wird (Peacock, J. et al., Biochim. Biophys. Acta 655, 243-250, 1981).

Das Homopolymer-tailing der dscDNA mit dGTP wurde unter gleichen Bedingungen ausgeführt, außer das ungefähr 100 ng der dscDNA pro Reaktion mit 30 Einheiten terminale Transferase für 30 Minuten bei 37$^°$C eingesetzt wurden. Die Reaktion wurde durch Zugabe von 50 $\mu$l 25 mM EDTA und durch 15-minütige Hitzeinaktivierung bei 70$^°$C beendet. Die analytische Reassoziation der dGTP-geschwänzten dscDNA mit dem dCTP-geschwänzten Vektor pUC9 erfolgte in 50 $\mu$l 10 mM Tris HCl pH 7.5, 1 mM EDTA, 150mM NaCl für 120 Minuten bei 58$^°$C. Die unterschiedlichen Verhältnisse von dscDNA zur Vektor-DNA machte man sich zunutzte, um die maximal erreichbare Anzahl Klone pro eingesetzter Menge an dscDNA zu ermitteln. Die Transformation erfolgte über CaCl$_2$ - kompetente E. coli RRI Zellen (Peacock, S. et al., 1981, loc. cit.),

indem 100 $\mu$l dieser Zellen mit 50 $\mu$l Reassoziations-Reaktionsmischung gemischt und diese danach auf LB-Platten, die 50 $\mu$g/ml Ampicillin enthielten, ausplattiert wurden. Dasjenige Verhältnis, das die höchste Transformationszahl ergab, wurde für "Scale-up" Experimente herangezogen, um die endgültige cDNA-Genbank festzulegen. Durch Einsetzen von 50 ng dscDNA sind ungefähr 20.000 unabhängige Klone erhalten worden.

Beispiel 5: Screening der U-937 cDNA Genbank für HLZ-Klone

a. Kolonie-Hybridisierung

Um positive, ein Fragment oder das komplette HLZ-Gen enthaltende Transformanten aufzufinden, erfolgte zuerst ein "Screening" der cDNA-Genbank über Kolonie-Hydridisierung. Bei denjenigen Transformanten, die positiv zu sein schienen, erfolgte ein weiteres Screening über "dot-blot"-Analyse.

Die Transformanten wurden über Nacht auf LB + Ampicillin (50 $\mu$g/ml)-Platten bei 37°C kultiviert. Die Kolonien wurden danach auf Nitrozellulosefilter (0.45 $\mu$m, Schleicher und Schuell) übertragen. Sowohl Platten als auch Filter wurden derart gekennzeichnet, daß sie die Identifikation positiver Transformanten ermöglichen würden. Die Filter wurden sorgfältig auf die Platten gelegt, dort für 20 Minuten bei Raumtemperatur (RT) belassen, dann abgehoben und auf 0,5 N NaOH/1.5 M NaCl-gesättigtem 3MM Filter (3 MM Chr, Whatman) plaziert (mit den Kolonien nach oben). Um einen NaOH-Überschuß zu entfernen, wurden die Nitrozellulose-Filter nach 15-minütiger Inhubation auf einen trockenen 3MM Filter transferriert und anschließend für 3 Minuten auf 3MM Filter belassen, der vorher mit 1 M Tris HCl pH 7 / 1.5 M NaCl getränkt worden war. Die Filter wurden danach für 20 Sekunden in 3 x SSC getaucht, luftgetrocknet und bei 80°C für 2 Stunden gebacken. Die derart präparierten Filter wurden über Nacht bei 65°C in 3 x SSC, 0,1 % SDS (Serval), 10 mM EDTA unter langsamen Schütteln vorgewaschen. Der Puffer wurde einige Male gewechselt. Das Vorwaschen wurde als abgeschlossen betrachtet, wenn keine erkennbaren Spuren von Bakterienkolonien auf den Filtern gefunden werden konnten. Unmittelbar nach dem Waschen wurden die Filter in 6 x SSC, 1 x Denhardt's Lösung, 0,5 % SDS, 100 $\mu$g/ml denaturierte Kalbs-Thymus DNA (Sigma), 0,05 % Natriumpyrophosphat vorhybridisiert. Nach 3-stündiger Inhubation bei 37°C wurden die Filter über Nacht bei 37°C mit (gamm - $^{32}$p) ATP markiertem 17-mer Ei-ON19 hybridisiert. Die Hybridisierungslösung enthielt 6 x SSC / 1 x Denhardt's Lösung, 20 $\mu$g/ml Hefe-tRNA (Typ XX, Sigma), 0,05 % Natriumpyrophosphat, mit Polynukleotid-Kinase endmarkiertes 17-mer Ei-ON19. Die Kinase-Reaktion ist in Beispiel 3 beschrieben. Nach der Hybridisierung wurden die Filter in 6 x SSC 0,05 % Natriumpyrophosphat wie folgt gewaschen: 3 mal für 5 Minuten bei RT, 30 Minuten bei 37°C, 10 Minuten bei 47°C und 10 Minuten bei 53°C (fakultativ). Die Filter wurden dann luftgetrocknet und ein Röntgenfilm (X-Omat RP, Kodak) mit Hilfe einer "Dupont intensifying screen" für 24 Stunden bei - 70°C geschwärzt. Nach der Entwicklung der Filme wurden 48 Kolonien, die zweifelsfrei stärkere Signale gegenüber dem Hintergrund gaben, für weitere Analysen (dot-blot-Analyse) ausgewählt.

b. Dot-blot-Hydridisierung

2 ml - Proben von 30 $\mu$g/ml Amipicillin enthaltendem LB wurden mit 48 ausgewählten Kolonien inokuliert. Die Kulturen wurden über Nacht bei 37°C unter kräftigem Schütteln kultiviert. Die DNA wurde nach der Methode von Birnboim und Doly (Birnboim, H.C. and Doly, J., Nucl. Acids Res. 7, 1513-1523, 1979) präpariert. Jedes DNA-Präparat wurde in 67.5 $\mu$l H$_2$O gelöst, 7.5 $\mu$l 4 N NaOH dazugegeben und anschließend für eine Stunde bei 65°C im Wasserbad inkubiert. Die Proben wurden danach in Eis gekühlt, 20 $\mu$l 1.5 N HCl und 195 $\mu$l 2 x SSC nacheinander dazugegeben. Das Gesamtvolumen jeder Probe betrug 290 $\mu$l. Portionen von 145 $\mu$l wurden über ein Bio-Dot Apparat (Bio-Rad) gefilter um die DNA auf zwei Nitrozellulosefilter zu fixieren. Vor dem Filtern wurden die Nitrozellulosefilter für 20 Minuten mit 2 x SSC getränkt. Nach dem Filtern wurden die Filter luftgetrocknet und für 2 Stunden bei 80°C gebacken. Ein Filter wurde mit (gamma-$^{32}$P) ATP-markiertem Ei-ON19 17-mer, ein anderer mit (gamma-$^{32}$P) ATP-markiertem Ei-ON20 17-mer hydridisiert. Die Hydridisierungs- und Waschbedingungen sind im Beispiel 5a beschrieben. Mit den Filtern wurde ein Kodak X-Omat RP Röntgenfilm für 6 Stunden bei - 70°C geschwärzt. Sechs Klone, die positive Hydridisationssignale zeigten, wurden identifiziert und die ursprünglichen Transformanten mit pHL2, pHL8, pH114-1, pHL21, pHL23 und pHL25 bezeichnet. Die Größe der cDNA-Insertionen in den ausgewählten Klonen wurde durch Restriktionsanalyse bestimmt. Die DNA der besagten Klone, die nach der Methode von Birnboim und Doly isoliert wurde, wurde mit BamHI und HindIII verdaut und in einem 0,8 %igen Agarose-Minigel für 4 Stunden (150 V) getrennt. Die Plasmide pHL14-1, pHL21 und pHL23 enthalten 500 bp lange Insertionen. Die Insertionen von pHL2 und pHL8 besitzen eine Größe von 300 bp.

Beispiel 6: Struktur und DNA-Sequenz der HLZ cDNA (Klon HL14-1)

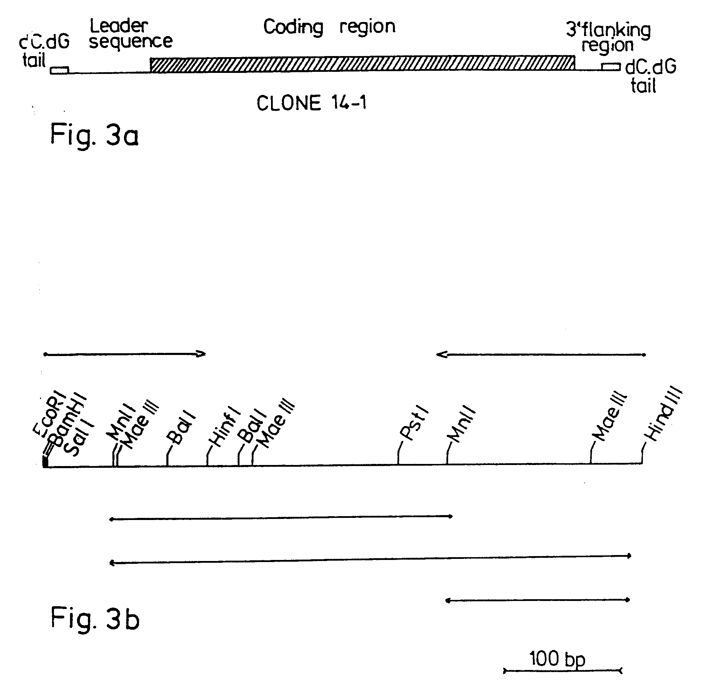

Einer der positiven Klone (Beispiel 5 b), als HL14-1 bezeichnet, enthält eine ungefähr 500 bp lange Insertion und wurde für eine detailierte Analyse ausgewählt. Die Struktur der HLZ cDNA zeigt Abbildung 3a.

20 μg pHL14-1 Plasmid DNA wurden vollständig mit den Restriktionsendonukleasen BamHI und HindIII verdaut, am 5'Ende mit (gamma-$^{32}$P) ATP radioaktiv markiert (Beispiel 3b) und danach mit einer zweiten Endonuklease gespalten. Die an einer Stelle markierten DNA-Fragmente wurden durch Polyacrylamid- oder Agarose-Gelelektrophorese getrennt. Markierte DNA-Fragmente wurden durch Elektroelution wiedergewonnen und nach der Methode von Maxam und Gilbert (Maxam, A.M. and Gilbert, W., Methods in Enzymology 65, 499-560, 1980) sequenziert. Die oberen Pfeile in Abbildung 3b zeigen Richtung und Ausdehnung der nach dieser Methode durchgeführten Sequenzanalyse an; die Sternchen bezeichnen radioaktiv markierte Stellen. Der interne Teil der pHL14-1 cDNA wurde nach der Methode von Sanger (Sanger, F. et al., Proc. Natl. Acad. Sci. USA 74, 5463-5467, 1977) sequenziert. Das HLZ cDNA enthaltende BamHI- HindIII Restriktionsfragment von pHL14-1 wurde partiell mit MnII-Restriktionsendonuklease verdaut, mit der Klenow-Polymerase nach der Methode von Maniatis et al (Maniatis, T., Fritsch, E.F., Sambrook, J., Molecular cloning, a Laboratory Manual, Cold Spring Harbor Laboratory, 1982) mit "blunt"-Enden versehen und im SmaI - geschnitten Vektor M 13 mp9 kloniert. Sowohl alle DNA-Manipulationen mit M 13 als auch die Sequenzierung wurden exakt nach der in der Amersham Broschüre "M 13 cloning and sequencing handbook" beschriebenen Methode durchgeführt. Die Restriktionsfragmente, die nach dieser Methode kloniert und sequenziert worden sind, zeigt Abbildung 3b (untere Pfeile). Angezeigt sind nur die Positionen relevanter Restriktionsorte im Klon HL14-1. Das Vorhandensein von Erkennungsstellen für die Endonukleasen MnII, MaeIII und Hinfl wurde experimentell bestätigt.

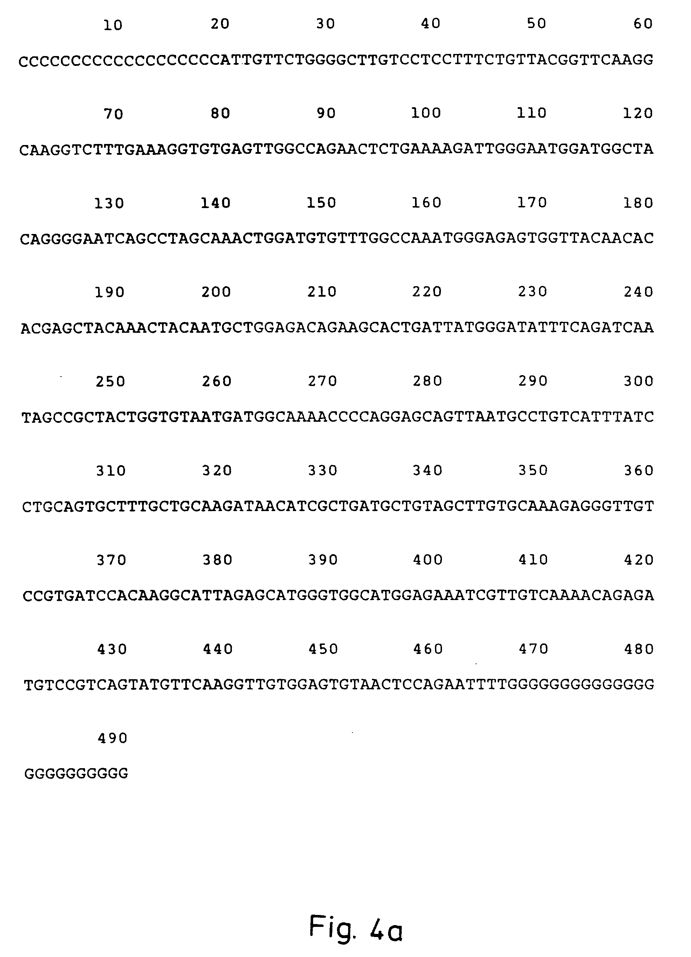

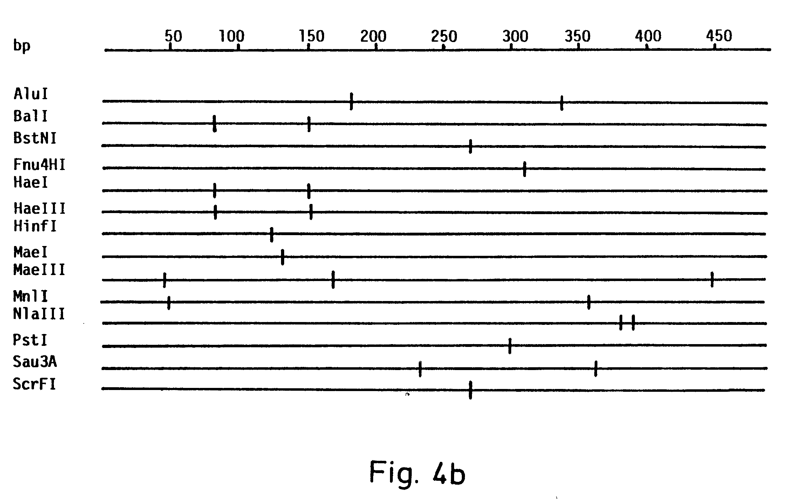

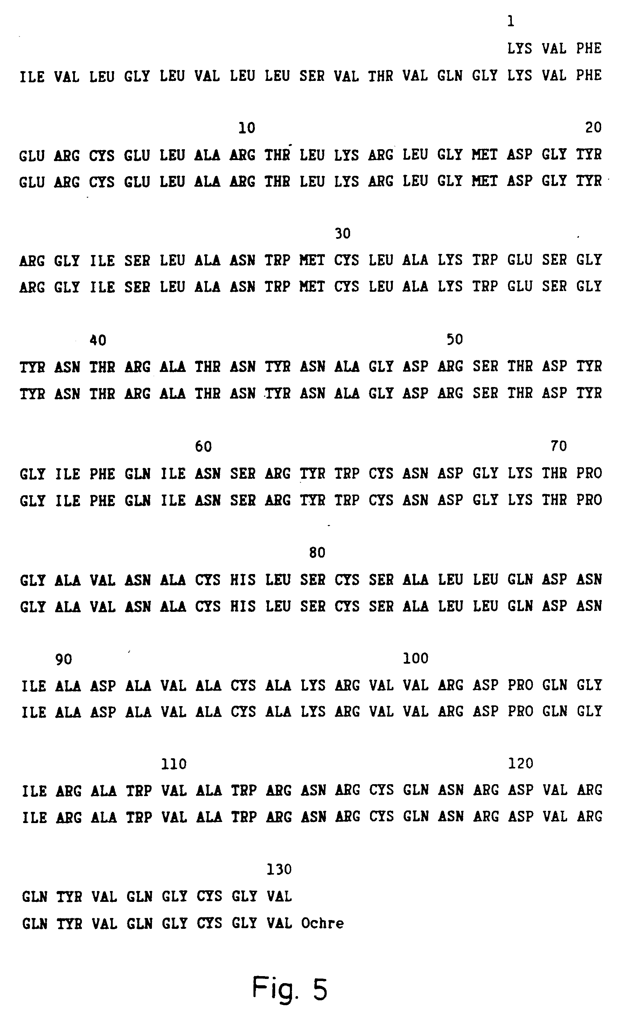

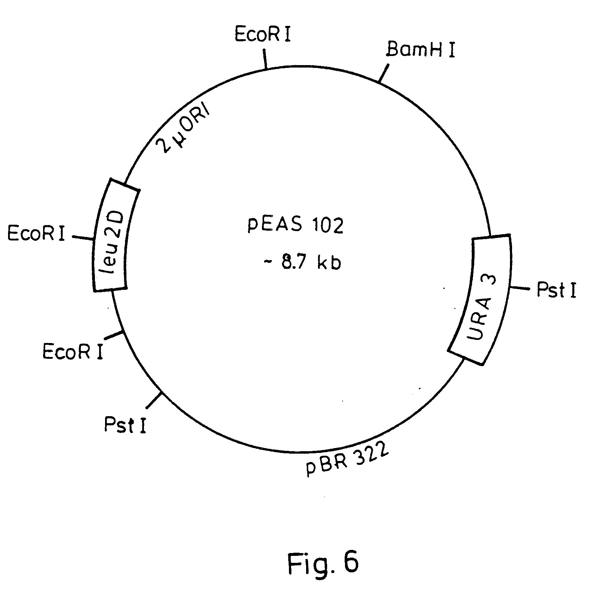

Das Ergebnis der DNA-Sequenzierung zeigt Figur 4a. Ab dem 20. Nukleotid stromaufwärts wird ein offener Leserahmen in der HLZ cDNA-Sequenz vorgefunden. Die Nukleotide 62-451 korrespondieren präzise mit der Aminosäuresequenz für Human-Lysozym (Figur 5), gefolgt durch das Translations-Terminationscodon TAA.

Die Nukeotide 20-61, die für 14 hauptsächlich hydrophobe Aminosäuren kodieren: 5' Ile Val Leu Gly Leu Val Leu Leu Ser Val Thr Val Gln Gly LYS VAL usw., codieren für einen Teil des HLZ-Leader- oder Signal-Peptids, ähnlich wie bei anderen sekretierten Proteinen (Lingappa, V.R. and Blobel, G., Recent. Prog. Horm. Res. 36, 415-475, 1980). Die Aminosäure-Zusammensetzung an der Verknüpfungsstelle ("splice junction") von Human-Prälysozym ist derjenigen von Hühnereiweiß-Lysozym sehr ähnlich (Gln Gly LYS VAL für Human Prälysozym gegenüber Leu Gly LYS VAL für Hühnereiweiß-Lysozym (Weisman L.S. et al, J. Biol. Chem., 261, 2309-2313, 1986).

Beispiel 7: Konstruktion von pHL14-23

Für die Konstruktion des Expressionsplasmids zur Herstellung von HLZ wurde das längste 5'Ende der HLZ-Insertion (Klon HL14-1, Plasmid pHL14-1) mit dem längsten 3'Ende der HLZ-Insertion (Klon HL23, Plasmid pHL 23) - Beispiel 5b - kombiniert. Das 3'-Ende der HLZ-DNA von pHL23 hat die Sequenz

```
        PstI   10            20            30            40
 5'   GACTGCAGTG    CTTTGCTGCA    AGATAACATC    GCTGATGCTG


            50            60            70            80
      TAGCTTGTGC    AAAGAGGGTT    GTCCGTGATC    CACAAGGCAT


            90           100           110           120
      TAGAGCATGG    GTGGCATGGA    GAAATCGTTG    TCAAAACAGA


                                               MaeIII
           130           140           150           160
      GATGTCCGTC    AGTATGTTCA    AGGTTGTGGA    GTGTAACTCC


           170           180           190           200
      AGAATTTTCC    TTCTTCAGCT    CATTTTGTCT    CTCTCACAAT


                                 MaeIII
           210           220           230           240
      TAAGGGAGTA    GGTTAAGTGA    AAGGTCACAT    ACCATTATTT


           250           260
      CGGGGGGGGG    GGGGGGGGGG    3'
```

Das 5'-Ende bis zur PstI-Stelle (Fig. 4a) entspricht der DNA-Sequenz von pHL 14-1. Es wurde ca. 1 $\mu$g von pHL23 mit PstI und HindIII bei 36°C (50 mM TrisHCl pH8.0, 10 mM MgCl$_2$, 50 mM NaCl) verdaut und in einem 1%igen Agarosegel aufgetrennt. Das aus dem Agarosegel nach der Methode von Dretzen, G. (Dretzen, G. et al. Anal. Biochem. 112, 295-298, 1981) isolierte 260 bp PstI/HindIII-Fragment enthält das 3'-Ende des HLZ-Strukturgens. Dieses Fragment (ca. 500 ng) wurde in pHL 14-1 (ca. 50 ng) ligiert nachdem das 190 bp PstI/HindIII-Fragment aus pHL 14-1 entfernt worden ist. Die Ligierung der DNA wurde in einem 20 $\mu$l Ansatz bei 14°C über Nacht in Ligationspuffer (66 mM TrisHCl pH7.6, 6.6 mM MgCl$_2$, 10mM DTT, 1mM rATP) sowie 1U T4 DNA Ligase durchgeführt und die Ligasemischung für die Transformation kompetenter Zellen von E.coli HB101 nach bekannten Methoden (Maniatis, T. et al. Molecular Cloning, Cold Spring Harbor Press, S. 220, 1982) eingesetzt.

Beispiel 8: Konstruktion einer Expressionskassette unter Kontrolle des ADHI-Promotors

a. Verknüpfung des ADHI-Promotors mit dem $\alpha$-Faktor-Leader

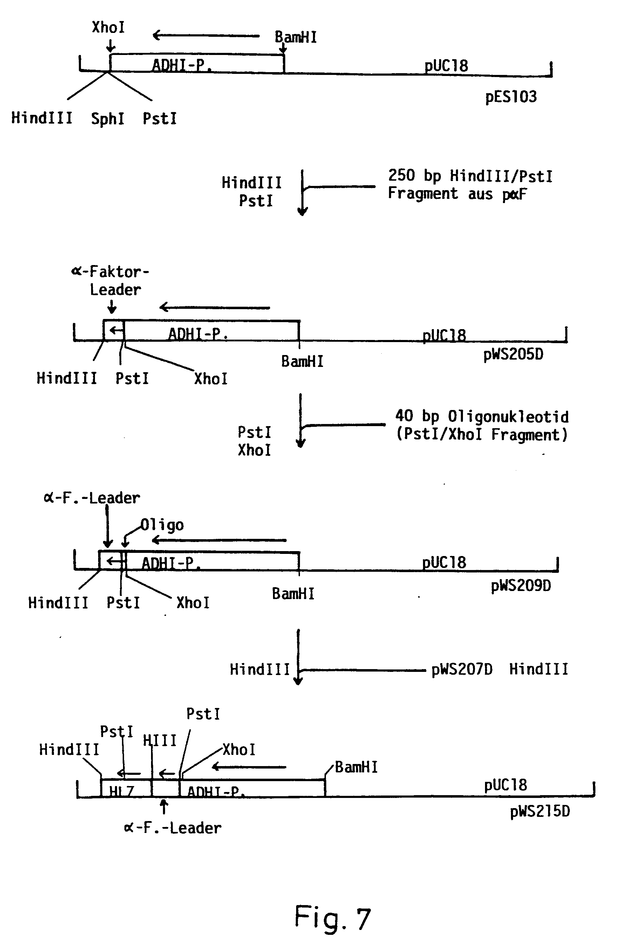

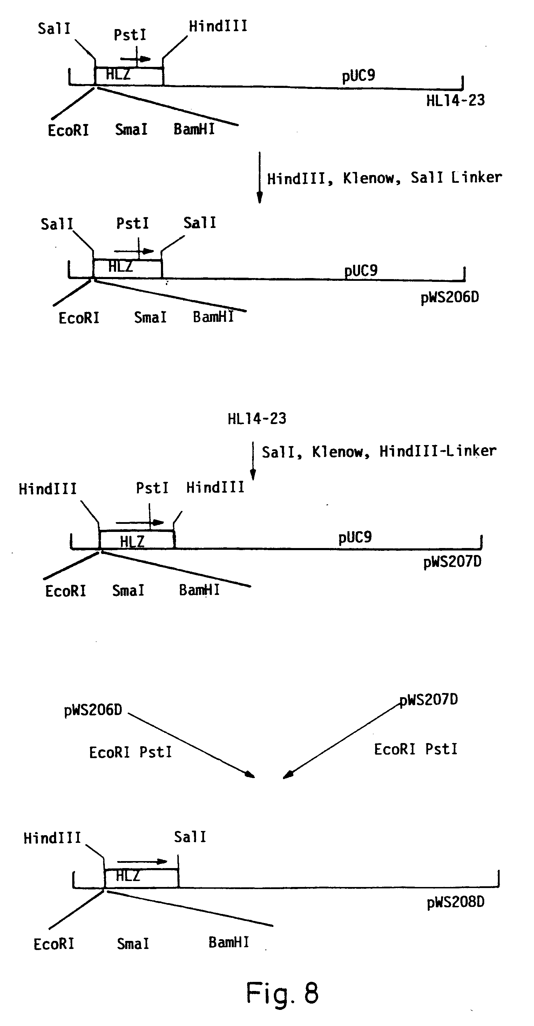

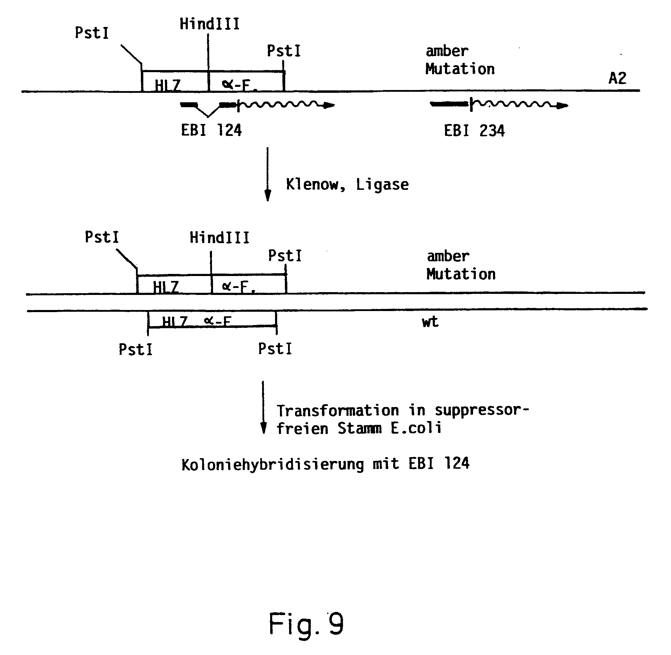

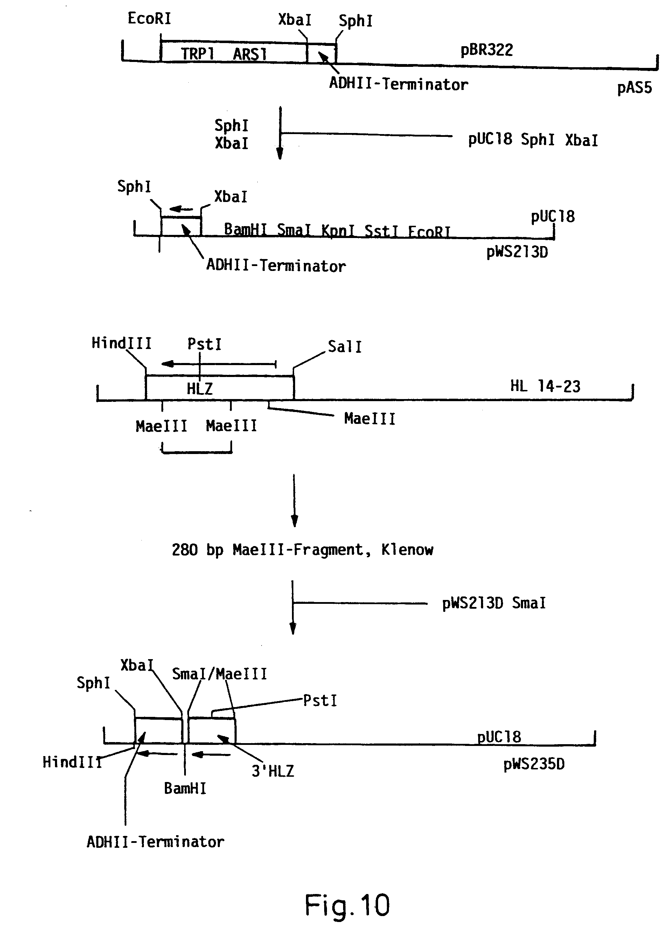

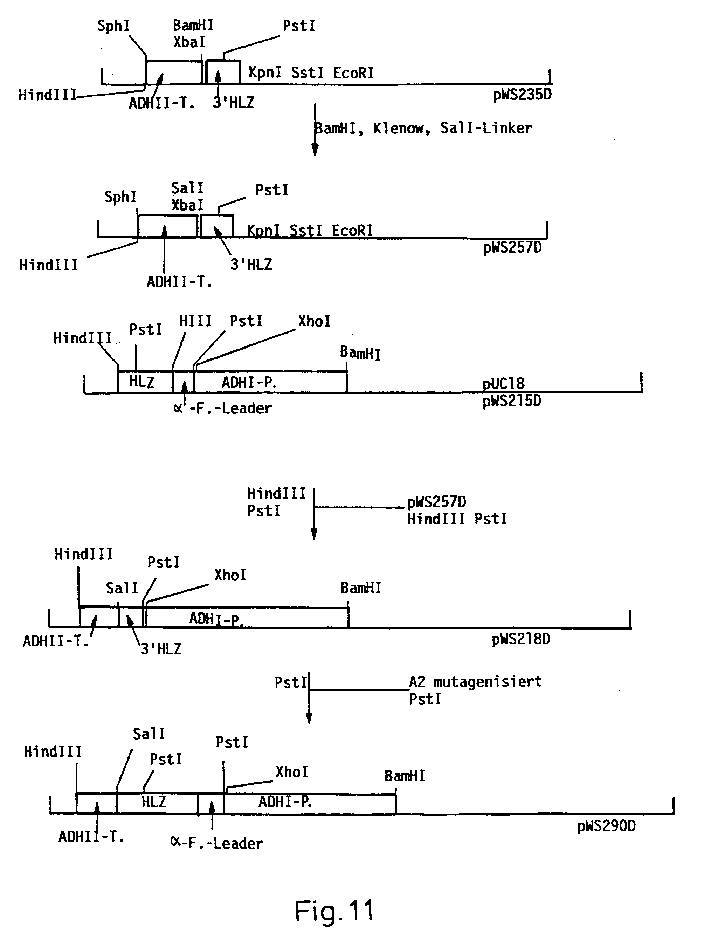

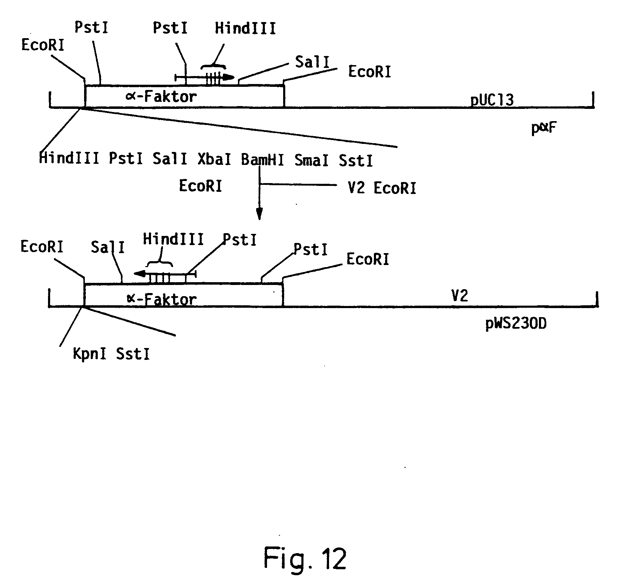

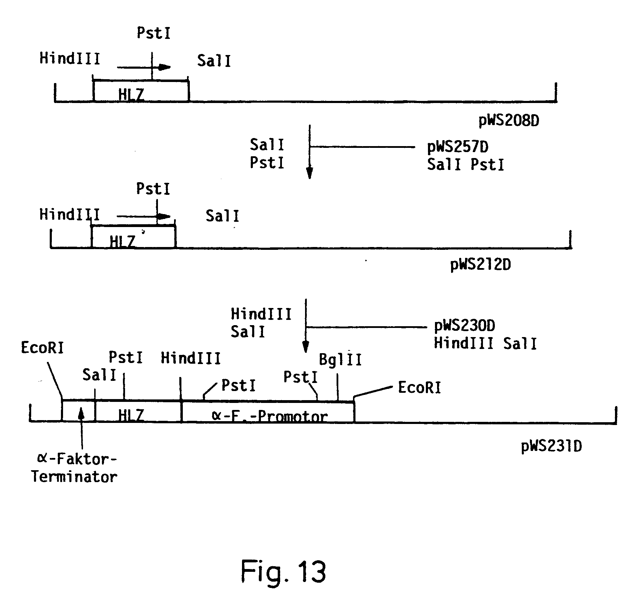

Ausgegangen wurde vom pUC18-Derivat pES103, das den ADHI-Promotor als 1.5 kb großen BamHI/XhoI-Fragment enthält. Anstatt pES103 kann auch YEp13 (ATCC 37 115) in gleicher Weise benutzt werden (Ammerer, G., Methods in Enzymology, 101, 192-201, 1983). Neben der XhoI-Stelle befinden sich eine PstI- und eine HindIII-Stelle, sodaß ein 250 bp großes HindIII/PstI-Fragment des $\alpha$-Faktor-Gens aus p$\alpha$F, das einen Großteil des $\alpha$-Faktor-Leaders enthält, (Fig.7), zwischen die HindIII- und die PstI-Stelle von pES103 einligiert werden kann. Die DNA-Sequenz des HindIII/PstI Fragments mit dem $\alpha$-Faktor-Leader ist publiziert (Singh, A. et al., Nucleic Acid Res. 11 (12), 4049-63, 1983).

Dazu wurden 1 $\mu$g pES103 und 5$\mu$g p$\alpha$F in 50 mM TrisHCl pH8.0, 10 mM MgCl$_2$, 50mM NaCl bei

36°C mit HindIII und PstI vollständig verdaut und wie oben beschrieben (Beispiel 7) ligiert, wobei ca. 50 ng pES103 (HindIII/PstI) und ca. 500 ng pαF (HindIII/PstI) eingesetzt wurden. Das entstandene Plasmid, in E.coli HB101 transformiert, mit der richtigen Orientierung des α-Faktor-Leaders zum ADHI-Promotor wurde pWS205D benannt. Die an der Verbindungsstelle zum ADHI-Promotor fehlenden 25 bp an α-Faktor-Leadersequenz samt dem ATG Startcodon wurde durch ein nach der Phosphotriester Methode (Efimov, V.A, et al., Nucleic Acid Res. 10, 6675-94, 1982) synthetisiertes 40-mer Oligonukleotid ersetzt. Zu diesem Zweck wurden 1 μg von pWS205D mit XhoI und PstI in 50 mM TrisHCl pH8.0, 10 mM MgCl₂, 50 mM NaCl bei 36°C vollständig verdaut. Das mit XhoI - und PstI-Enden versehene Oligonuleotid

```
5'        TCGAGAAAAGAATGAGATTTCCTTCAATTTTTACTGCA      3'
3'             CTTTTCTTACTCTAAAGGAAGTTAAAAATG            5'
     XhoI                                          PstI
```

wurde mit XhoI/PstI geschnittenem pWS205D ligiert: die Oligonukleotide wurden in einer Konzentration von 10 pMol/μl Wasser aufgenommen, jeweils 5 μl der Lösungen der beiden DNA-Stränge wurden vereinigt, 10 min bei 65°C erhitzt und nach Abkühlung auf Raumtemperatur wurden 1 μl von dieser Lösung zur Ligasereaktion in Ligasepuffer und unter den Bedingungen, wie unter Beispiel 7 beschrieben, eingesetzt. Das entstandene Plasmid pWS209D enthält somit die ca. 1.5 kb lange Region mit dem ADHI-Promotor und dem kompletten α-Faktor-Leader bis zum Beginn der codierenden Region des α-Faktors.

b. Verknüpfung des α-Faktor-Leaders mit dem HLZ-Gen

Da die α-Faktor-Leader Sequenz in pWS209D mit einer HindIII-Stelle endet und das HLZ-Gen in pHL14-23 am 3'-Ende von einer HindIII- und am 5'-Ende von einer SalI-Stelle begrenzt wird, wurde pH14-23 mit SalI in 6mM TrisHCl pH8.0, 6 mM MgCl₂, 150 mM NaCl bei 36°C vollständig verdaut und die überstehenden Enden mit Klenow-Polymerase (Gesamtvolumen von 25 μl in Ligasepuffer wie bei Beispiel 7, 0,5 μg geschnittene DNA, je 100 μM dATP, dTTP, dCTP, dGTP, 1 U Klenow-Polymerase, 30 min, Raumtemperatur) aufgefüllt. Die anschließende Reinigung der stumpfendigen SalI Verdauungsprodukte erfolgte im 1%igen Agarosegel. Daraufhin erfolgte die Linkerligation mit einem synthetischen HindIII-Linker (New England Biolabs) (Maniatis, T. et al., Molecular Cloning, Cold Spring, Harbor Press, S. 316, 1982). Die Linker wurden in einer Konzentration von 1 μg/μl Wasser aufgenommen. Die Kinasierung des HindIII-Linkers erfolgte in einem Reaktionsgemisch aus 1 μl 10x Linkerkinasierungspuffer (0,66 M TrisHCl pH 7,9, 10 mM ATP, 10 mM Spermidin, 0,1 M MgCl₂, 150 mM DTT, 2 mg/ml BSA), 1 μl Linker, 6 μl H₂O sowie 2 U T4 DNA Kinase bei 37°C für 1 Stunde. Die Linkerligation mit dem kinasierten HindIII-Linker wurde mit ca. 0.5 μg/μl des mit SalI und Klenow-Polymerase behandelten pHL14-23 in 10 μl 1 x Linkerkinasierungspuffer mit 10 U T4 DNA Ligase bei 14°C über Nacht durchgeführt. Mit dem erhaltenen Plasmid pWS207D, in dem das HLZ-Gen jetzt von zwei HindIII-Stellen flankiert wird, wurde E.coli HB101 transformiert.

Nach HindIII-Verdauung von 1 μg pWS207D (50 mM TrisHCl pH 8,0, 10 mM MgCl₂, 50 mM NaCl, 36°C) konnte jetzt das HLZ-Gen in HindIII geschnittenem pWS209D (5 μg) als HindIII-Fragment hinter den α-Faktor-Leader einligiert werden. Für die Ligasereaktion wurden nach Auftrennung und Isolierung der HindIII-Fragmente in 1%igem Agarosegel (Dretzen et al., siehe oben) ungefähr 50 ng von pWS207D und etwa 500 ng von pWS209D in einem 20 μl Ansatz, bestehend aus Ligationspuffer (66 mM TrisHCl pH 7,6, 6.6 mM MgCl₂, 10 mM DTT, 1mM rATP) und 1 U T4 DNA Ligase eingesetzt. Die Reaktion erfolgte bei 14°C über Nacht. Das entstandene Plasmid wurde pWS215D benannt, welches das HLZ-Gen in der richtigen Orientierung zum α-Faktor-Leader bzw. zum ADHI-Promotor enthält.

c. Exakter Übergang zwischen α-Faktor-Leader und HLZ-Gen

Für die Herstellung eines exakten N-Terminus des HLZ-Gens ist es notwendig, daß die Proteaseschnittstelle, die für die Reifung des α-Faktors verantwortlich ist, exakt vor der Sequenz für die erste Aminosäure des reifen HLZ positioniert wird. Somit mußten die von der Konstruktion der cDNA abstammenden überschüssigen Nukleotide (ca. 20 dG-dC-Paare) sowie derjenige Anteil an Nukleotiden, der für die authentische HLZ-Leadersequenz codiert, entfernt werden.

Plasmid pWS215D wurde mit PstI vollständig verdaut (50 mM TrisHCl pH 8,0, 10 mM MgCl₂, 50 mM NaCl, 36°C) und ein 500 bp großes Fragment, welches einen Großteil des α-Faktor-Leaders sowie das 5'-Ende des HLZ-Gens enthält, aus einem 1%igen Agarosegel isoliert (Dretzen et al. siehe oben), nach Carter, P. et al. in M13 amp18-am4 rekloniert (Carter, P., Bedoulle, H., Winter, G., Nucleic Acid Res.13, 4431-43, 1985) und in den E.coli Stamm TGI transformiert.

Ein rekombinanter Phage, der die gewünschte Orientierung aufwies, A2, wurde als Ausgangsmaterial der Einzelstrang-DNA für die in vitro Mutagenese benutzt.

Die Template-DNA wurde wie für die Didesoxy-Sequenzierung präpariert (M13 cloning and sequencing handbook, Amersham International plc, Amersham UK). Ein 20-mer Oligonukleotid EBI124, welches den gewünschten Übergang zwischen α-Faktor-Leader und HLZ-Gen enthält, und ein 17-mer Selektionsoligonukleotid EBI234, welches die im M13-Vektor vorhandene Ambermutation korrigiert, wurden nach der Phosphotriester-Methode synthetisiert (Efimov, v.A, et al., Nucleic. Acid Res. 10, 6675-94, 1982), über präparative Polyacrylamidgelelektrophorese gereinigt und als Primer für die Synthese des zweiten DNA-Stranges verwendet. Die Oligonukleotid-Mutagenese wurde im Wesentlichen nach der Methode von Zoller, M.J. und Smith, M. DNA 3, 479-488, 1984, durchgeführt: 150 pmol des Selektionsoligonukleotids EBI234 der Sequenz

5'        **AAGAGTCTGTCCATCAA**        3'

und 150 pmol des mutagenen Primers EBI124 der Sequenz

5'        **GGATAAAAGAAAGGTCTTTG**        3'

wurden phosphoryliert mit 4 U T4 Polynukleotid-Kinase (New England Biolabs) in 20 μl 50 mM TrisHCl pH 8.0, 10 mM MgCl$_2$, 1 mM rATP, 5 mM DTT bei 37°C für 45 min und anschließend bei 70°C für 10 min erhitzt. Die kinasierten Primer, je 7.5 pmol, wurden gegen 0,5 pmol Template-DNA in 10 μl 10 mM TrisHCl pH 8.0, 10 mM MgCl$_2$, 50 mM NaCl hybridisiert, wobei für 3 min auf 100°C erhitzt und anschließend über die Dauer von 1 Stunde auf Raumtemperatur abgekühlt wurde. Das renaturierte Gemisch wurde in Eis abgekühlt, das Volumen auf 20 μl 10 mM TrisHCl pH 8.0, 10 mM MgCl$_2$, 25 mM NaCl, 0,25 mM dNTP (dATP, dTTP, dCTP, dGTP) 0,25 mM rATP eingestellt und die Primer mit 1 U des großen Fragments der DNA Polymerase I (Biolabs) in Anwesenheit von 10 U T4 DNA Ligase (Biolabs) extendiert. Die Reaktion wurde für 16 Stunden bei 14°C durchgeführt. Kleine Aliquote des Gemisches von 0.1, 1.5 und 10 μl wurden dann dazu benutzt, kompetente Zellen von E-coli HB2154 (supressorlos) zu transfizieren, während der Zellrasen mit HB2155 hergestellt wurde (Carter P., et al. Nucleic Acids Res. 13, 4431-4443, 1985)

Von den 222 anhand der transfektierten DNA erhaltenen Plaques wurden 84 auf eine LB-Platte übertragen, bis sich Kolonien infizierter Bakterien entwickelten (37°C, 16 Stunden). Ein "Nitrozellulose-blot" wurde präpariert und mit dem $^{32}$P-markierten mutagenen Oligonukleotid EBI124 gescreent. Das Nitrozellulosefilter wurde in 6xSSPE (0,9 M NaCl, 0,06 M NaH$_2$PO$_4$, 6mM EDTA) für 5 min bei Raumtemperatur vorgewaschen, in Hybridisierungspuffer (6xSSPE, 1% Sarkosyl [Sigma], 100 μg/ml zufällig gespaltene tRNA) für 15 min bei 37°C vorhybridisiert und bei Raumtemperatur (22°C) für 16 Stunden in Hybrisierungspuffer mit 0,4 x 10$^6$ cpm/ml Probe hybridisiert. Die Probe wurde mit (γ$^-$$^{32}$P) ATP wie folgt 5' endmarkiert: 30 pmol des mutagenen Primers EBI124 wurden mit 4 U T4 Polynukleotidkinase (Biolabs) in 20 μl 50 mM TrisHCl pH 7.6, 10 mM MgCl$_2$, 10 mM DTT, 1 mM Spermidin, 100 μg/ml BSA und 10 pmol (γ$^-$$^{32}$P) ATP (5000 Ci/mmol, Amersham) bei 37°C für 45 min phosphoryliert. Das Reaktionsgemisch wurde danach an Biogel P-6DG (Biorad) in TE-Puffer (10 mM TrisHCl pH 7.5, 1 mM EDTA) chromatographisch gereinigt, um nicht-eingebautes (γ$^-$$^{32}$P) ATP von dem $^{32}$P-markierten Oligonukleotiden zu trennen.

Nach der Hybridisierung wurden die Filter 3 mal in 6xSSC (0,9 M NaCl, 0,09 M Na-Citrat) bei Raumtemperatur für 5 min und 1mal in vorgewärmten 6xSSC bei 37, 47,5, 50 und 56°C für 5 min gewaschen und nach jedem Waschvorgang autoradiographiert. Die bei 56°C (T$_D$+2°C) durchgeführte Autoradiographie machte einen Klon sichtbar, A2/25, der gegen das mutagene Oligonukleotid stark hybridisierte.

Der mutmaßliche Mutantenphage wurde Plaque- gereinigt, nochmals zur Identifizierung von reinen Mutantenphagen gescreent und, um die Deletion zu verifizieren, sequenziert.

Doppelsträngige DNA aus Klon A2/25 wurde nach Yanisch-Perron et al. (Yanisch-Perron et al., Gene 33, 103-119, 1985) präpariert und mit PstI gespalten (ca. 5 μg in 50 mM TrisHCl pH 8.0, 10 mM MgCl$_2$, 50 mM NaCl, 36°C). Das 440 bp PstI-Fragment, das jetzt den gewünschten Übergang zwischen α-Faktor-Leader und reifem HLZ aufweist, wurde aus einem 1%igen Agarosegel wie beschrieben (Dretzen et al. s.o.)isoliert und für die unten näher erläuterten Ligationen (Beispiel 8e und 9d) eingesetzt.

d. ADHII-Terminator und 3'-Ende des HLZ-Gens

Der ADHII-Terminator (Beier, D.R., Young, E.T,. Nature 300, 724-728, 1982; Russell, D.W. et al., J.Biol. Chem. 258, 2674-2682, 1983) wurde aus dem Plasmid pAS5 auf die Weise isoliert, daß 5 $\mu$g von pAS5 mit SphI/XbaI verdaut (6 mM TrisHCl pH 7.4, 6 mM MgCl$_2$, 150 mM NaCl, 1 mM DTT, 36°C) und wie beschrieben aus einem 1%igen Agarosegel isoliert wurde. Die DNA-Sequenz des ADHII-Terminators ist von Russell, D.W. et al. (siehe oben) beschrieben. Die Verdauung von pUC18 (Vieira, J. Messing, J., Gene 19, 259-268, 1982; Yanisch-Perron et al. Gene 33, 103-119, 1985) wurde mit SphI und XbaI unter den oben angegebenen Bedingungen durchgeführt. Anschließend wurden ca. 50 ng des verdauten Vektors und ca. 500 ng des ADHII-Terminators (SphI/XbaI-Fragment) in einem Reaktionsansatz von 20 $\mu$l in Ligationspuffer (66 mM TrisHCl pH 7.6, 6.6 mM MgCl$_2$, 10 mM DTT, 1 mM rATP) mit 1 U T4 DNA-Ligase bei 14°C über Nacht ligiert. Mit dem erhaltenen Plasmid pWS213D wurde E.coli HB101 transformiert.

Da die HLZ-DNA aus pHL14-23 nach dem Stopcodon noch weitere Nukleotide einer nichtcodierenden Region, sowie eine Strecke von 20 dC-dG-Paaren aufweist, die von der Herstellung der cDNA stammen, wurden diese entfernt. Zu diesem Zweck wurde pHL14-23 mit MaeIII verdaut (20 mM TrisHCl pH 8.0, 6 mM MgCl$_2$, 350 mM NaCl, 1 mM DTT, 45°C) und isoliert (Dretzen et al. siehe oben). Das erhaltene 280 bp große MaeIII-Fragment wurde exakt im Stopcodon geschnitten und enthält das 3'-Ende des HLZ-Gens.

Die überstehenden Enden wurden mit Klenow-Polymerase aufgefüllt, indem ca. 0,5 $\mu$g der mit MaeIII geschnittenen DNA in einem Gesamtvolumen von 25 $\mu$l Ligasepuffer (siehe oben) mit je 100 $\mu$M dATP, dTTP, dCTP, dGTP und 1 U Klenow-Polymerase für 30 min bei Raumtemperatur zur Reaktion gebracht wurden. Ca. 1 $\mu$g pWS213D wurde mit SmaI (6 mM TrisHCl pH 8.0, 6 mM MgCl$_2$, 20 mM KCl) bei 36°C verdaut und das stumpfendige MaeIII Fragment von pHL14-23 (ca. 500 ng) in die SmaI-Stelle von pWS213D (ca. 50 ng) ligiert (Bedingungen wie oben beschrieben).

Das entstandene Plasmid mit der richtigen Orientierung des HLZ-Gens zum ADHII-Terminator wurde pWS235D benannt. Dieses Plasmid enthält das 3'-Ende des HLZ-Gens bis einschließlich des Stopcodons und anschließend den ADHII-Terminator. Zwischen dem HLZ-Gen und dem ADHII-Terminator befinden sich noch eine BamHI- und eine XbaI-Schnittstelle, die von der Multiklonierungsstelle des Plasmid pUC18 stammen. Da die BamHI-Stelle bei weiteren Konstruktionen stört, wurde sie entfernt. Dazu wurde pWS235D mit BamHI bei 36°C geschnitten (6 mM TrisHCl pH 8.0, 6 mM MgCl$_2$, 150 mM NaCl, 1 mM DTT), die überstehenden Enden mit Klenow-Polymerase aufgefüllt (siehe oben) und ein SalI- Linker nach den schon beschriebenen Bedingungen (Beispiel 8b) eingebaut.

Das entstandene Plasmid wurde pWS257D benannt und damit E.coli HB101 transformiert. Die Verbindungssequenz zwischen dem 3'-Ende vom HLZ-Gen und dem 5'-Ende des ADHII-Terminators lautet

```
5'HLZ--GTAAC GGG GGATC GGTCGACC GATCC TCTAGA--ADHII-T.3'
       --CATTG CCC CCTAG CCAGCTGG CTAGG AGATCT--
              1     2    3       4      5      6
```

1 = 3'-Ende von HLZ, mit MaeIII verdaut und mit Klenow-Polymerase aufgefüllt, mit dem Stopcodon TAA

2 = Restanteil der SmaI Klonierungsstelle (pWS213D)

3 = Geöffnete und mit Klenow-Polymerase aufgefüllte BamHI-Stelle

4 = SalI-Linker (New England Biolabs)

5 = Anderes Ende der BamHI-Stelle von 3 (geöffnet und mit Klenow-Polymerase aufgefüllt)

6 = XbaI-Stelle und Start des ADHII-Terminators

e. Konstruktion der Expressionskassette

Das unter Beispiel 8b konstruierte Plasmid pWS215D (ca. 1 $\mu$g) wurde mit PstI und HindIII verdaut (Bedingungen wie unter Beispiel 8a), wodurch mit dem erhaltenen 4.2 kb-Fragment das gesamte HLZ-Gen sowie ein Großteil des $\alpha$-Faktor-Leaders herausgeschnitten wird. Unter den selben Bedingungen wurde pWS257D mit PstI und HindIII verdaut und das erhaltene Fragment mit dem ADHII-Terminator und dem 3'-Ende des HLZ-Gens in die PstI/HindIII-Stelle einligiert. Die Ligasereaktion war wie oben beschrieben (Beispiel 8d). Im erhaltenen Plasmid pWS218D liegen direkt am Beginn des $\alpha$-Faktor-Leaders das 3'-Ende des HLZ-Gens, welches mit dem Stopcodon endet, sowie daran anschließend der ADHII-Terminator. Zur Fertigstellung der Expressionskassette fehlen noch ein Großteil des α-Faktor-Leaders und das 5'-Ende des HLZ-Gens, sowie der korrekte Übergang zwischen diesen beiden Elementen. Daher wurde das unter Beispiel 8c hergestellte mutagenisierte 440 bp große PstI-Fragment (ca 500 ng) in die PstI-Schnittstelle von pWS218D (ca. 50 ng) unter den oben beschriebenen Bedingungen ligiert. Das Plasmid mit der richtigen Orientierung des 440 bp großen PstI-Fragments (5'-Ende des HLZ-Gens und Großteil des α-Faktor-Leaders (3'-Ende)) zum ADHI-Promotor bzw. zum 3'-Ende des HLZ-Gens wurde pWS290D benannt. Das Plasmid pWS290D enthält somit alle beschriebenen Elemente in der richtigen Reihenfolge und Orientierung zueinander, sowie die modifizierten, exakten Übergänge zwischen den einzelnen Elementen: 1450 bp ADHI-Promotor, 260 bp α-Faktor-Leader (endet mit einer Sequenz, die für eine Proteaseschnittstelle codiert), 390 bp HLZ-Gen (beginnt mit einem Lysincodon mit anschließender Sequenz für das mature HLZ-Protein und endet mit dem Stopcodon des HLZ-Gens), 330 bp ADHII-Terminator.

f. Expression von HLZ in Hefe

Die Expressionskassette pWS290D (5μg) wurde mit HindIII und BamHI bei 36°C verdaut (50 mM TrisHCl pH 8.0, 10 mM $MgCl_2$, 50 mM NaCl) und in den ebenfalls mit HindIII und BamHI geschnittenen Multicopyvector pJDB207 (1μg) ligiert. Sowohl die Expressionskassette pWS290D (5 ng) als auch der Multicopyvektor pJDB207 (1μg) (Beggs, J.D., Gene cloning in yeast, in : Williamson, R. (Ed.), Genetic engineering, Vol.2, Academic Press, London 1981, 175-203; DSM 3181) wurden mit HindIII und BamHI bei 36°C verdaut (50 mM TrisHCl pH 8.0, 10 mM $MgCl_2$, 50 mM NaCl). In das so geöffnete Multicopyplasmid pJDB207 (ca. 50 ng) wurde das präparierte HindIII/BamHI-Fragment (ca. 500 ng) ligiert (20 μl Ansatz mit Ligationspuffer und unter Bedingungen wie bei Beispiel 7 angegeben mit 1 U T4 DNA Ligase). Mit diesem Plasmid 129/29 wurden verschiedene heterothallische Hefestämme von Saccharomyces cerevisiae wie folgt transformiert (Beggs, J.D. Nature 275, 104-109, 1978): Aus einer über Nacht in YPD (1% Bacto yeast extract, 2% Bacto pepton, 2% Glucose) angezüchteten Vorkultur wurden $10^7$ Zellen/ml in 50 ml YPD inokuliert und 2 Generationen bei 28°C (im allgemeinen für 3 Stunden) kultiviert. Bei 5000 rpm wurde für 5 min abzentrifugiert, die Zellen in 5 ml SED (1 M Sorbit, 25 mM EDTA pH 8.0, 50 mM DTT) resuspendiert und 10 min bei 30°C langsam geschüttelt. Bei 1600 rpm wurde 5 min abzentrifugiert, in 5 ml SCE (1 M Sorbit, 0,1 M $Na_3$ Citrat, pH 5.8, 10 mM EDTA) resuspendiert, davon 100 μl entnommen und in 2,9 ml $H_2O$ suspendiert. Die Ausbildung von Sphäroplasten wurde bei 600 nm gemessen. Daraufhin erfolgte eine Behandlung mit 50 μl Glusulase unter langsamen Schütteln bei 28°C. Nach verschiedenen Zeiten wurden wieder 100 μl Sphäroplasten entnommen, in 2.9 ml $H_2O$ suspendiert und die $E_{600}$ bestimmt.

Sowie die Extinktion bei 600 nm auf ca. 30% des Ausgangswertes abgesunken war (meist nach 20 bis 30 min.) wurden die Zellen bei 1600 rpm abzentrifugiert (Tischzentrifuge). Die Sphäroplasten wurden einmal mit 5 ml CaS (1 M Sorbit, 10 mM $CaCl_2$, 10 mM TrisHCl pH 7.5) gewaschen, wieder langsam abzentrifugiert und in 0.5 ml CaS aufgenommen.

Aliquote Mengen an Sphäroplasten wurden entnommen, 1 μg Plasmid-DNA (129/29) dazugegeben (bei größeren Volumen an DNA-Lösung muß diese auf 1 M Sorbit eingestellt werden), 15 min. bei Raumtemperatur stehengelassen und 1 ml PEG (20% w/v PEG 4000, 10 mM $CaCl_2$, 10 mM TrisHCl ph 7.5, steril filtriert) dazugegeben. Es wurde 15 min. bei Raumtemperatur stehengelassen, bei 1600 rpm abzentrifugiert (Tischzentrifuge), das Pellet in 150 μl SOS (1 M Sorbit, 33% v/v YPD, 6.5 mM $CaCl_2$, 13.5 μg der filtersterilisierten Aminosäure, auf die selektioniert wird) resuspendiert und 20 min. bei 30°C stehengelassen. Dieses Gemisch wurde in 3 ml bei 45°C vorgekühltem Topagar (1 M Sorbit, 2.5% Agar, 0.67% BYNB wo aa [Bacto yeast nitrogen base ohne Aminosäuren], 2% Glucose, 1% 100 x Aminosäure-Mix) leicht geschüttelt, auf Bottomagarplatten (2% Agar, 0.67% BYNB wo aa, 2% Glucose, Aminosäuremix wie bei Topagar) gegossen und 2-4 Tage bei 30°C kultiviert. Der 100 x Aminosäuremix -leu enthielt je 2 g/l Adeninsulfat, Arginin, Uracil, Asparagin, Glutamin, Histidin, Methionin, Lysin, Tyrosin, Phenylalanin, Valin, Threonin, Tryptophan. Transformierte Hefestämme (Saccharomyces cerevisiae) wurden auf sc-leu (0.67% Bacto yeast nitrogen base ohne Aminosäuren, 5% Glucose, 1% 100 x Aminosäuremix -leu) bei 30°C gezüchtet.

Die folgende Tabelle zeigt die Ergebnisse der Lysozymexpression. Die Mengenwerte an Lysozym wurden durch spezifische Antikörperbindung an Lysozym (Elisa-Test) ermittelt.

| Transformant | Stamm | Lysozym (mg/l Kultur) |
|---|---|---|
| 1/29 | WS21-5 | 0.4 |
| 1/31 | WS21-5 | 0.31 |
| 2/30 | WS21-3 | 0.89 |
| 2/31 | WS21-3 | 0.42 |
| 6/30 | WS21-1 | 1.0 |
| 6/31 | WS21-1 | 1.2 |

```
WS21-1 =  a   leu2 his3 trpl pep4
WS21-3 = α   leu2 his3 ura3 pep4
WS21-5 = α   leu2 his3 trpl argl pep4
```

Beispiel 9: Konstruktion einer Expressionskassette unter Kontrolle des α-Faktor-Promotors

Publizierte Arbeiten (Bitter, G. A. et al. Proc. Natl. Acad. Sci. USA 81, 5330-34, 1984; Brake, A. J. et al. Proc. Natl. Acad. Sci. USA 81, 4642-46, 1984) beschreiben die Insertion von heterologen Genen zwischen die HindIII- und die SalI-Stelle des α-Faktor-Gens. Dabei wirkt die Sequenz zwischen der EcoRI- und der HindIII-Stelle als Promotor und als Leaderpeptid, die Sequenz zwischen SalI und EcoRI als Transkriptionsterminator.

a. Klonierung des α-Faktors in einen geeigneten Vektor

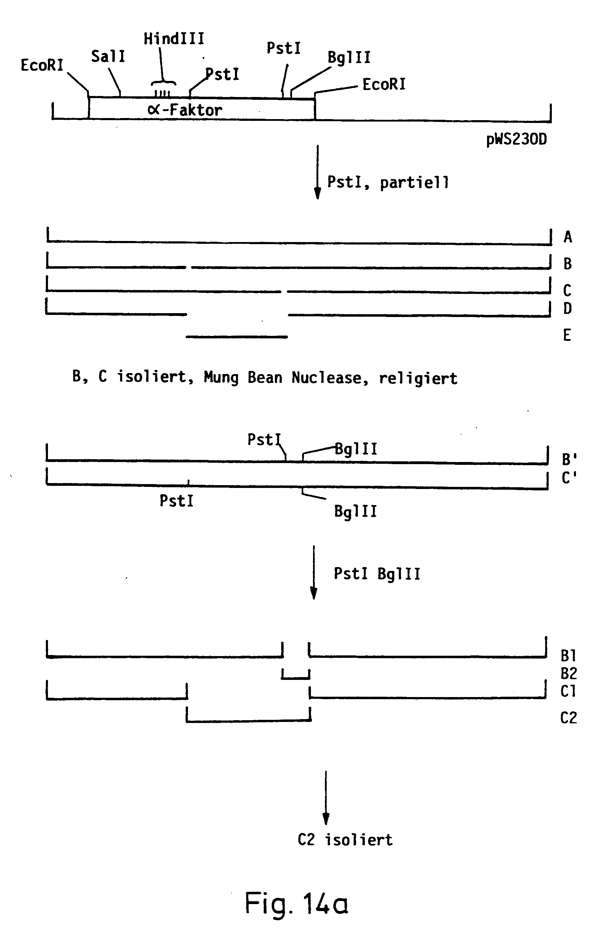

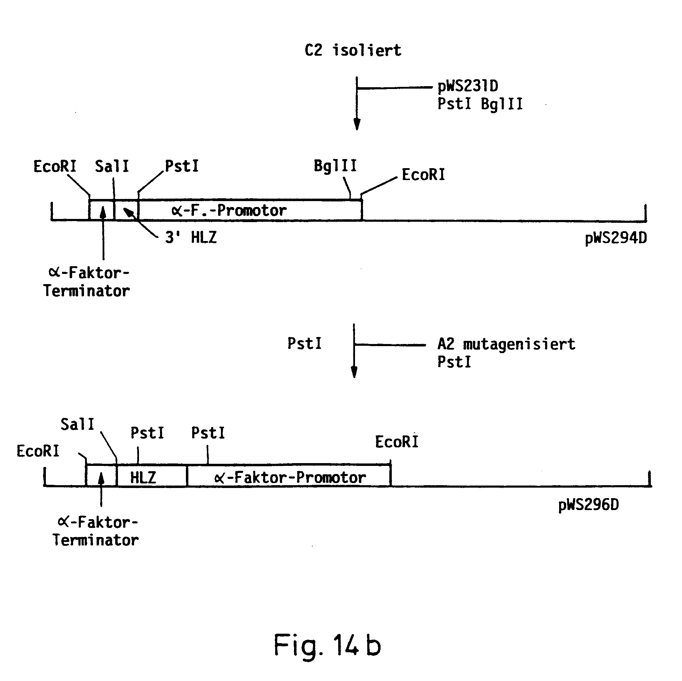

Das α-Faktor-Gen wurde aus dem pUC13-Derivat pαF (5 μg) als EcoRI-Fragment isoliert (100 mM TrisHCl pH 7.5, 5 mM MgCl₂, 50 mM NaCl, 1 mM DTT, 36°C) und in den Vektor V2 kloniert. Der Vektor V2 wurde wie folgt konstruiert: Es wurde pUC18 mit SamI und HindIII gespalten, die Enden wie schon beschrieben mit Klenow-Polymerase aufgefüllt und der verbliebene Anteil von pUC18 religiert, sodaß der entstandene Vektor V2 nur noch drei (EcoRI, SstI, KpnI) der ursprünglich 10 Klonierungsstellen trägt. Somit enthält Vektor V2 keine HindIII- und SalI-Stelle mehr, sodaß sich die einzigen Schnittstellen für diese beiden Enzyme im α-Faktor-Gen befinden. Der Vektor V2 (1 μg) wurde mit EcoRI verdaut und ca. 500 ng EcoRI-verdautes pαF in ca. 50 ng EcoRI-verdauten V2 ligiert (Ligasereaktion wie unter Beispiel 7 beschrieben). Das entstandene und in E. coli HB101 transformierte Plasmid pWS230D wurde für weitere Konstruktionen eingesetzt (Beispiel 9b, c). Das PstI/BglII-Fragment aus Plasmid pWS230D (Fig. 14a) enthält den ungefähr 900 bp langen Promotor. Die daneben liegende PstI-Stelle liegt etwa 25 bp innerhalb des translatierten Bereiches. Ungefähr 25 bp vor der PstI-Stelle, die zu den vier HindIII-Stellen benachbart liegt sowie weiter stromaufwärts, befinden sich die für die Transkription des α-Faktor-Gens notwendigen DNA-Regionen. Die Leader-Sequenz des α-Faktors beginnt etwa 25 bp links von dieser zweiten PstI-Stelle (ATG) und endet ziemlich exakt an der ersten der vier HindIII-Stellen. Die Länge beträgt ca. 280 bp. Der Terminator liegt zwischen der SalI- und EcoRI-Stelle.

b. Verbindung des HLZ-Gens mit dem α-Faktor-Gen

Damit das HLZ-Gen in der richtigen Orientierung zum α-Faktor-Promotor bzw. -Terminator eingebaut werden kann, mußten die Restriktionsschnittstellen HindIII und SalI, die pHL14-23 flankieren, vertauscht werden. Dazu wurden ca. 5 μg von pHL14-23 mit SalI (6 mM TrisHCl pH 8.0, 6 mM MgCl₂, 150 mM NaCl, 36°C) bzw. HindIII (50 mM TrisHCl pH 8.0, 10 mM MgCl₂, 50 mM NaCl, 36°C) geschnitten, die überstehenden Enden jeweils mit Klenow-Polymerase (0.5 μg geschnittene DNA in 25 μl Reaktionsvolumen wie unter Beispiel 8b) aufgefüllt und das Reaktionsgemisch in einem 1%igen Agarosegel gereinigt. Ein HindIII-Linker (New England Biolabs) wurde in die SalI-(pWS207D) und ein SalI-Linker (New England Biolabs) in die HindIII-Stelle (pWS206D) einligiert (je ca. 0.5 $\mu$g/$\mu$l der DNA-Fragmente), wie unter Beispiel 8b beschrieben, und in E. coli HB101 transformiert. Die entstandenen Plasmide pWS206D und pWS207D (je ca. 5 $\mu$g) wurden mit EcoRI und PstI geschnitten (50 mM TrisHCl pH 8.0, 10 mM MgCl$_2$, 50 mM NaCl, 36°C) und unter den Bedingungen der oben beschriebenen Ligasereaktion (Beispiel 7) aneinanderligiert, wobei Plasmid pWS208D erhalten wurde. Auch hier weist das unter Beispiel 8d beschriebene 3'-Ende des HLZ-Gens das Problem auf, daß nach dem Stopcodon weitere Nukleotide vorhanden sind. Aus diesem Grund wurde das 3'-Ende des HLZ-Gens wie folgt bis zum Stopcodon verkürzt: Plasmid pWS257D (Konstruktion von Beispiel 8d) enthält das 3'-Ende des HLZ-Gens bis zum Stopcodon, daran anschließend eine SalI-Stelle sowie den ADHII-Terminator, der jedoch für diese Konstruktion ohne Bedeutung ist. Die Plasmide pWS208D und pWS257D wurden mit SalI und PstI vollständig verdaut (PstI in 50 mM TrisHCl pH 8.0, 10 mM MgCl$_2$, 50 mM NaCl, 36°C; 1 Stunde, danach Erhöhung der NaCl-Konzentration auf 150 mM und SalI dazugegeben) und in einem 1%igen Agarose gereinigt und isoliert (Dretzen et al. siehe oben). Das SalI/PstI-Fragment von pWS257D, welches das 3'-Ende des HLZ-Gens enthält, ersetzt das SalI/PstI-Fragment aus pWS208D, indem das Fragment von pWS257D (ca. 500 ng) in die SalI/PstI-Stelle von pWS208D (ca. 50 ng) wie oben beschrieben ligiert wurde. Das entstandene Plasmid pWS212D enthält nun das komplette HLZ-Gen als HindIII/SalI-Fragment, wobei das HLZ-Gen am 3'-Ende mit dem Stopcodon endet. Die Plasmide pWS212D und pWS230D (Beispiel 9a) wurden mit HindIII und SalI vollständig verdaut (HindIII in 50 mM TrisHCl pH 8.0, 10 mM MgCl$_2$, 50 mM NaCl, 36°C, 1 Stunde, danach Erhöhung der NaCl-Konzentration auf 150 mM und SalI dazugegeben), sodaß das aus pWS212D herausgeschnittene HLZ-Gen als HindIII/SalI-Fragment (ca. 500 ng) (Isolierung aus einem 1%igen Agarosegel wie oben beschrieben) in den mit HindIII/SalI geöffneten Vektor (ca. 50 ng) einligiert werden konnte (Ligasereaktion wie unter Beispiel 7 beschrieben). In dem entstandenen Plasmid pWS231D liegt das HLZ-Gen jetzt in der richtigen Orientierung zum $\alpha$-Faktor-Promotor und -Terminator.

c. Entfernung einer PstI-Stelle aus dem $\alpha$-Faktor-Gen

Das $\alpha$-Faktor-Gen enthält 2 PstI-Stellen. Die nicht im kodierenden Bereich gelegene ist für die weitere Konstruktion sehr hinderlich, sodaß diese durch folgende Vorgangsweise entfernt wurde: Plasmid pWS230D (Konstruktion siehe Beispiel 9a) wurde mit PstI partiell geschnitten, indem 10 $\mu$g pWS230D in 50 mM TrisHCl pH 8.0, 10 mM MgCl$_2$, 50 mM NaCl mit 10 U PstI für 10 min. bei 36°C verdaut wurden. Aus einem 1%igen Agarosegel wurden diejenigen Fragmente isoliert, die genau einen Schnitt aufwiesen (Fragmente B und C in Abbildung 14a). Zum Wegverdauen der 3'-überstehenden Enden mit Mung Bean Nuclease wurde ungefähr 1 $\mu$g der so erhaltenen linearisierten DNA von pWS230D in 13 $\mu$l H$_2$O aufgenommen und 2 $\mu$l 10 mM ZnCl$_2$, 1 $\mu$l 4 M NaCl, 2 $\mu$l 0,3 M Natriumacetat pH 4.75, 2 $\mu$l Mung Bean Nuclease (300 U, Pharmacia) dazugegeben. Dieses Gemisch wurde für 15 min. bei Raumtemperatur stehengelassen und anschließend direkt auf ein 1%iges Agarosegel aufgetragen und die Fragmente aus dem Gel isoliert (Dretzen et al. siehe oben). Anschließend erfolgte Religation der Fragmente B und C (Bedingungen wie unter Beispiel 7 angegeben), wobei die PstI-Stelle, die bei der partiellen Verdauung geschnitten worden war, zerstört wurde und die jeweils nicht geschnittene erhalten blieb.

Auf diese Weise konnte ein Plasmid isoliert werden, in dem die gewünschte PstI-Stelle nicht mehr vorhanden war und die andere jedoch erhalten blieb (C'). Sowohl dieses Plasmid (C') als auch pW5231D wurden mit PstI und BgIII vollständig verdaut (50 mM TrisHCl pH 8.0, 10 mM MgCl$_2$, 50 mM NaCl, 36°C). Das isolierte C2-Fragment (850bp) aus C' (ca. 500 ng) wurde in PstI/BgIII verdautem pWS231D (ca. 50 ng) einligiert (Ligasereaktion wie unter Beispiel 7 beschrieben), sodaß Plasmid pWS294D erhalten wurde. Dieses Plasmid pWS294D enthält jetzt ein $\alpha$-Faktor-Gen mit einer PstI-Stelle, in welches das 3'-Ende des HLZ-Gens in der richtigen Orientierung insertiert ist.

d. Konstruktion der Expressionskassette

Plasmid pWS294D enthält den $\alpha$-Faktor-Promotor und den Anfang des $\alpha$-Faktor-Peptides bis zur PstI-Schnittstelle (ca. 20 bp Leaderpeptid) sowie daran anschließend das 3'-Ende des HLZ-Gens ab der PstI-Stelle bis zum Stopcodon. Zur vollständigen Expressionskassette fehlen noch das 3'-Ende des $\alpha$-Faktor-Leaders, das 5'-Ende des HLZ-Gens sowie der korrekte Übergang zwischen diesen beiden Elementen. Plasmid pWS294D (ca 1 $\mu$g) wurde mit PstI vollständig verdaut (50 mM TrisHCl pH 8.0, 10 mM MgCl$_2$, 50 mM NaCl, 36°C) und das mutagenisierte 440 bp PstI-Fragment aus dem Plasmid A2/25 (Beispiel 8c) (ca. 500 ng) entsprechend Beispiel 8e in die PstI-Stelle von pWS294D einligiert.
Das erhaltene Plasmid pWS296D enthält die Expressionskassette bestehend aus 900 bp Promotorsequenz vom $\alpha$-Faktor-Gen, 260 bp $\alpha$-Faktor-Leader (identisch mit der unter Beispiel 8e beschriebenen Länge der Sequenz), 390 bp HLZ-Gen (identisch mit der unter Beispiel 8e beschriebenen Länge der Sequenz), 290 bp α-Faktor-Terminator.

**Patentansprüche**

1. DNA-Molekül bestehend aus
   a) einer Nukleotidsequenz der Formel

5´AAGGTCTTTGAAAGGTGTGAGTTGGCCAGAACTCTGAAAAGATTGGGAATGGATGG

CTACAGGGGAATCAGCCTAGCAAACTGGATGTGTTTGGCCAAATGGGAGAGTGGTTAC

AACACACGAGCTACAAACTACAATGCTGGAGACAGAAGCACTGATTATGGGATATTTC

AGATCAATAGCCGCTACTGGTGTAATGATGGCAAAACCCCAGGAGCAGTTAATGCCTG

TCATTTATCCTGCAGTGCTTTGCTGCAAGATAACATCGCTGATGCTGTAGCTTGTGCA

AAGAGGGTTGTCCGTGATCCACAAGGCATTAGAGCATGGGTGGCATGGAGAAATCGTT

GTCAAAACAGAGATGTCCGTCAGTATGTTCAAGGTTGTGGAGTG,

   oder deren allele Variationen mit der Codierungsfunktion von Human-Lysozym
   und
   b) einer Hefe-Leadersequenz, welche mit dem 5'-Ende von a) verbunden ist.

2. DNA-Molekül umfassend eine Nukleotidsequenz, welche mit einem DNA-Molekül nach Anspruch 1 unter stringenten Bedingungen, die für mehr als 85 % Homologie selektieren, hydridisiert und für Human-Lysozym codiert.

3. DNA-Molekül nach Anspruch 2, dadurch gekennzeichnet, daß die stringenten Bedingungen der Hydridisierung für mehr als 90 % Homologie selektieren.

4. DNA-Molekül nach Ansprüchen 1 bis 3, dadurch gekennzeichnet, daß das 5' Ende des Moleküls ein ATG Triplett aufweist und das 3' Ende mit einem Stopcodon endet.

5. DNA-Molekül mit einer Nucleotidsequenz der Formel

5´ATTGTTCTGGGGCTTGTCCTCCTTTCTGTTACGGTTCAAGGCAAGGTCTTTGAAAG

GTGTGAGTTGGCCAGAACTCTGAAAAGATTGGGAATGGATGGCTACAGGGGAATCAGC

CTAGCAAACTGGATGTGTTTGGCCAAATGGGAGAGTGGTTACAACACACGAGCTACAA

ACTACAATGCTGGAGACAGAAGCACTGATTATGGGATATTTCAGATCAATAGCCGCTA

CTGGTGTAATGATGGCAAAACCCCAGGAGCAGTTAATGCCTGTCATTTATCCTGCAGT

GCTTTGCTGCAAGATAACATCGCTGATGCTGTAGCTTGTGCAAAGAGGGTTGTCCGTG

ATCCACAAGGCATTAGAGCATGGGTGGCATGGAGAAATCGTTGTCAAAACAGAGATGT

CCGTCAGTATGTTCAAGGTTGTGGAGTGTAACTCCAGAATTTTCCTTCTTCAGCTCAT

TTTGTCTCTCTCACAATTAAGGGAGTAGGTTAAGTGAAAGGTCACATACCATTATTTC

6. Rekombiniertes DNA-Molekül, zusammengesetzt aus einer vektoriellen Nukleotidsequenz und einem DNA-Molekül nach einem der Ansprüche 1 bis 5.

7. Rekombiniertes DNA-Molekül nach Anspruch 6, dadurch gekennzeichnet, daß es ein Expressionsvektor ist.

**8.** Rekombiniertes DNA-Molekül nach Anspruch 7, dadurch gekennzeichnet, daß der Expressionsvektor zusammengesetzt ist aus den Elementen Promotor, Translationsinitiationssignal, einem DNA-Molekül nach einem der Ansprüche 1 bis 5 und einem Terminator.

**9.** Rekombiniertes DNA-Molekül nach Anspruch 8, dadurch gekennzeichnet, daß der Promotor der ADHI-Promotor oder der α-Faktor Promotor, die Leadersequenz der α-Faktor-Leader und der Terminator der ADHII-Terminator oder der α-Faktor-Terminator ist.

**10.** Transformierte Wirtszelle, welche ein DNA-Molekül nach einem der Ansprüche 1 bis 5 oder ein rekombiniertes DNA Molekül nach einem der Ansprüche 6 bis 9 enthält.

**11.** Transformierte Wirtszelle nach Anspruch 10, dadurch gekennzeichnet, daß sie eine eukaryotische Zelle ist.

**12.** Transformierte Wirtszelle nach Anspruch 11, dadurch gekennzeichnet, daß sie eine Hefezelle ist.

**13.** DNA-Oligonukleotide der Formeln

$$5' \quad CC^A_GTC^A_GTT^A_GCACCA^A_GTA \quad 3'$$

$$5' \quad A^{AA}_{GG}CACATCCA^A_GTT^{^A}_{C{}_{G}}TGC \quad 3'$$

**14.** Verfahren zur Herstellung von Human-Lysozym, gekennzeichnet durch die Schritte
a. Isolierung einer Human-Lysozym mRNA aus der Gesamt-RNA aus einer Human-Zellinie
b. Konstruktion einer cDNA aus dem in Schritt a. gewonnenen Isolat und anschließende Klonierung der doppelsträngigen cDNA
c. Screening der aus Schritt b. erhaltenen cDNA Genbank über Hybridisierung mittels geeigneter Oligonukleotide,
d. Konstruktion der kompletten für Human-Lysozym codierenden cDNA mit der Nukleotidsequenz folgender Formel

5´AAGGTCTTTGAAAGGTGTGAGTTGGCCAGAACTCTGAAAAGATTGGGAATGGATGG

CTACAGGGGAATCAGCCTAGCAAACTGGATGTGTTTGGCCAAATGGGAGAGTGGTTAC

AACACACGAGCTACAAACTACAATGCTGGAGACAGAAGCACTGATTATGGGATATTTC

AGATCAATAGCCGCTACTGGTGTAATGATGGCAAAACCCCAGGAGCAGTTAATGCCTG

TCATTTATCCTGCAGTGCTTTGCTGCAAGATAACATCGCTGATGCTGTAGCTTGTGCA

AAGAGGGTTGTCCGTGATCCACAAGGCATTAGAGCATGGGTGGCATGGAGAAATCGTT

GTCAAAACAGAGATGTCCGTCAGTATGTTCAAGGTTGTGGAGTG

oder deren allele Variationen mit der Codierungsfunktion von Human-Lysozym

oder einer Nukleotidsequenz, welche mit den Formeln der vorstehenden Nukleotidsequenzen unter stringenten Bedingungen, die für mehr als 85 % Homologie selektieren, hybridisieren und für Human-Lysozym codieren,
e. Verbinden einer aus Schritt d. erhaltenen cDNA an ihrem 5' Ende mit einer Hefe-Leadersequenz,

f. Rekombination der aus Schritt e. erhaltenen DNA mit einer vektoriellen DNA und Konstruktion eines Expressionsplasmids, welches im wesentlichen zusammengesetzt ist aus den Elementen Promotor, Translationsinitiationssignal, einem aus Schritt e. erhaltenem DNA-Molekül, einem Stopcodon und einem Terminator,

g. Transformation einer geeigneten Wirtszelle mit einem aus Schritt f. erhaltenen rekombinierten DNA-Molekül, Kultivierung dieser Zellen in einem geeigneten Medium und Isolierung des erhaltenen Human-Lysozyms aus dem Medium.

**Claims**

1. DNA molecule consisting of
   a) a nucleotide sequence of the Formula

5´AAGGTCTTTGAAAGGTGTGAGTTGGCCAGAACTCTGAAAAGATTGGGAATGGATGG
CTACAGGGGAATCAGCCTAGCAAACTGGATGTGTTTGGCCAAATGGGAGAGTGGTTAC
AACACACGAGCTACAAACTACAATGCTGGAGACAGAAGCACTGATTATGGGATATTTC
AGATCAATAGCCGCTACTGGTGTAATGATGGCAAAACCCCAGGAGCAGTTAATGCCTG
TCATTTATCCTGCAGTGCTTTGCTGCAAGATAACATCGCTGATGCTGTAGCTTGTGCA
AAGAGGGTTGTCCGTGATCCACAAGGCATTAGAGCATGGGTGGCATGGAGAAATCGTT
GTCAAAACAGAGATGTCCGTCAGTATGTTCAAGGTTGTGGAGTG,

   or the allelic variations thereof with the coding function of human lysozyme
   and
   b) a yeast leader sequence connected to the 5' end of a).

2. DNA molecule comprising a nucleotide sequence with hybridises with a DNA molecule according to claim 1 under stringent conditions, selecting for more than 85% homology and which codes for human lysozyme.

3. DNA molecule according to claim 2, characterised in that the stringent conditions of hybridisation select for more than 90% homology.

4. DNA molecule according to claims 1 to 3, characterised in that the 5' of the molecule has an ATG triplet and the 3' end finishes with a stop codon.

5. DNA molecule with a nucleotide sequence of the Formula

5´ATTGTTCTGGGGCTTGTCCTCCTTTCTGTTACGGTTCAAGGCAAGGTCTTTGAAAG
GTGTGAGTTGGCCAGAACTCTGAAAAGATTGGGAATGGATGGCTACAGGGGAATCAGC
CTAGCAAACTGGATGTGTTTGGCCAAATGGGAGAGTGGTTACAACACACGAGCTACAA
ACTACAATGCTGGAGACAGAAGCACTGATTATGGGATATTTCAGATCAATAGCCGCTA
CTGGTGTAATGATGGCAAAACCCCAGGAGCAGTTAATGCCTGTCATTTATCCTGCAGT
GCTTTGCTGCAAGATAACATCGCTGATGCTGTAGCTTGTGCAAAGAGGGTTGTCCGTG
ATCCACAAGGCATTAGAGCATGGGTGGCATGGAGAAATCGTTGTCAAAACAGAGATGT
CCGTCAGTATGTTCAAGGTTGTGGAGTGTAACTCCAGAATTTTCCTTCTTCAGCTCAT
TTTGTCTCTCTCACAATTAAGGGAGTAGGTTAAGTGAAAGGTCACATACCATTATTTC

6. Recombined DNA molecule, made up of a vectorial nucleotide sequence and a DNA molecule according to one of claims 1 to 5.

7. Recombined DNA molecule according to claim 6, characterised in that it is an expression vector.

8. Recombined DNA molecule according to claim 7, characterised in that the expression vector is made up the elements promoter, translation initiation signal, a DNA molecule according to one of claims 1 to 5 and a terminator.

9. Recombined DNA molecule according to claim 8, characterised in that the promoter is the ADHI promoter or the α-factor promoter, the leader sequence is the α-factor leader and the terminator is the ADHII terminator or the α-factor terminator.

10. Transformed host cell which contains a DNA molecule according to one of claims 1 to 5 or a recombined DNA molecule according to one of claims 6 to 9.

11. Transformed host cell according to claim 10, characterised in that it is a eukaryotic cell.

12. Transformed host cell according to claim 11, characterised in that it is a yeast cell.

13. DNA oligonucleotides of the Formulae

$$5'\quad CC\overset{A}{\underset{G}{}}TC\overset{A}{\underset{G}{}}TT\overset{A}{\underset{G}{}}CACA\overset{A}{\underset{G}{}}TA\quad 3'$$

$$5'\quad A\overset{AA}{\underset{GG}{}}CACATCCA\overset{A}{\underset{G}{}}TT\overset{\overset{A}{T}}{\underset{\underset{G}{C}}{}}GC\quad 3'$$

14. Process for preparing human lysozyme, characterised by the steps:
a. Isolating a human lysozyme mRNA from the total RNA of a human cell line
b. Constructing a cDNA from the isolated material obtained in step a. and subsequently cloning the double stranded cDNA
c. Screening the cDNA library obtained from step b. by hybridisation by using suitable oligonucleotides,
d. Constructing the complete cDNA coding for human lysozyme with the nucleotide sequence of the following formula

5´AAGGTCTTTGAAAGGTGTGAGTTGGCCAGAACTCTGAAAAGATTGGGAATGGATGG
CTACAGGGGAATCAGCCTAGCAAACTGGATGTGTTTGGCCAAATGGGAGAGTGGTTAC
AACACACGAGCTACAAACTACAATGCTGGAGACAGAAGCACTGATTATGGGATATTTC
AGATCAATAGCCGCTACTGGTGTAATGATGGCAAAACCCCAGGAGCAGTTAATGCCTG
TCATTTATCCTGCAGTGCTTTGCTGCAAGATAACATCGCTGATGCTGTAGCTTGTGCA
AAGAGGGTTGTCCGTGATCCACAAGGCATTAGAGCATGGGTGGCATGGAGAAATCGTT
GTCAAAACAGAGATGTCCGTCAGTATGTTCAAGGTTGTGGAGTG

or the allelic variations thereof with the coding function of human lysozyme
or a nucleotide sequence, which hybridises with the formulae of the above mentioned nucleotide sequences under stringent conditions selecting for more than 85% homology, and code for a human lysozyme,

e. Linking of a cDNA obtained in step d. by the 5' end thereof with a yeast leader sequence,

f. Recombining the DNA obtained from step e. with a vectorial DNA and constructing an expression plasmid, which is essentially made up of the elements promoter, translation initiation signal, an DNA molecule obtained from step e, a stop codon and a terminator,

g. Transforming a suitable host cell with a recombined DNA molecule obtained from step f, cultivating these cells in a suitable medium and isolating the resulting human lysozyme from the medium.

**Revendications**

1. Molécule d'ADN consistant en
   a) une séquence nucléotidique de formule

5´AAGGTCTTTGAAAGGTGTGAGTTGGCCAGAACTCTGAAAAGATTGGGAATGGATGG

CTACAGGGGAATCAGCCTAGCAAACTGGATGTGTTTGGCCAAATGGGAGAGTGGTTAC

AACACACGAGCTACAAACTACAATGCTGGAGACAGAAGCACTGATTATGGGATATTTC

AGATCAATAGCCGCTACTGGTGTAATGATGGCAAAACCCCAGGAGCAGTTAATGCCTG

TCATTTATCCTGCAGTGCTTTGCTGCAAGATAACATCGCTGATGCTGTAGCTTGTGCA

AAGAGGGTTGTCCGTGATCCACAAGGCATTAGAGCATGGGTGGCATGGAGAAATCGTT

GTCAAAACAGAGATGTCCGTCAGTATGTTCAAGGTTGTGGAGTG,

   ou en ses variations alléliques avec la fonction de codage du lysozyme humain
   et
   b) une séquence de tête de levure qui est reliée à l'extrémité 5' de a).

2. Molécule d'ADN comprenant une séquence nucléotidique qui s'hybride avec une molécule d'ADN selon la revendication 1 dans des conditions strictes qui opèrent une sélection pour plus de 85% d'homologie et qui code pour le lysozyme humain.

3. Molécule d'ADN selon la revendication 2, caractérisée en ce que les conditions strictes de l'hybridation opèrent une sélection pour plus de 90% d'homologie.

4. Molécule d'ADN selon les revendications 1 à 3, caractérisée en ce que l'extrémité 5' de la molécule présente un triplet ATG et l'extrémité 3' se termine par un codon d'arrêt.

5. Molécule d'ADN comportant une séquence nucléotidique de formule

5´ATTGTTCTGGGGCTTGTCCTCCTTTCTGTTACGGTTCAAGGCAAGGTCTTTGAAAG

GTGTGAGTTGGCCAGAACTCTGAAAAGATTGGGAATGGATGGCTACAGGGGAATCAGC

CTAGCAAACTGGATGTGTTTGGCCAAATGGGAGAGTGGTTACAACACACGAGCTACAA

ACTACAATGCTGGAGACAGAAGCACTGATTATGGGATATTTCAGATCAATAGCCGCTA

CTGGTGTAATGATGGCAAAACCCCAGGAGCAGTTAATGCCTGTCATTTATCCTGCAGT

GCTTTGCTGCAAGATAACATCGCTGATGCTGTAGCTTGTGCAAAGAGGGTTGTCCGTG

ATCCACAAGGCATTAGAGCATGGGTGGCATGGAGAAATCGTTGTCAAAACAGAGATGT

CCGTCAGTATGTTCAAGGTTGTGGAGTGTAACTCCAGAATTTTCCTTCTTCAGCTCAT

TTTGTCTCTCTCACAATTAAGGGAGTAGGTTAAGTGAAAGGTCACATACCATTATTTC

**6.** Molécule d'ADN recombinée, constituée par une séquence nucléotidique vectorielle et une molécule d'ADN selon l'une des revendications 1 à 5.

**7.** Molécule d'ADN recombinée selon la revendication 6, caractérisée en ce qu'il s'agit d'un vecteur d'expression.

**8.** Molécule d'ADN recombinée selon la revendication 7, caractérisée en ce que le vecteur d'expression est constitué par les éléments promoteur, signal d'initiation de la traduction, une molécule d'ADN selon l'une des revendications 1 à 5 et un terminateur.

**9.** Molécule d'ADN recombinée selon la revendication 8, caractérisée en ce que le promoteur est le promoteur ADHI ou le promoteur de facteur $\alpha$, la séquence de tête est la séquence de tête de facteur $\alpha$ et le terminateur est le terminateur ADHII ou le terminateur de facteur $\alpha$.

**10.** Cellule hôte transformée, qui contient une molécule d'ADN selon l'une des revendications 1 à 5 ou une molécule d'ADN recombinée selon l'une des revendications 6 à 9.

**11.** Cellule hôte transformée selon la revendication 10, caractérisée en ce qu'il s'agit d'une cellule eucaryotique.

**12.** Cellule hôte transformée selon la revendication 11, caractérisée en ce qu'il s'agit d'une cellule de levure.

**13.** Oligonucléotides d'ADN de formules

$$5' \quad CC^ATC^ATT^ACACCA^ATA \quad 3'$$
$$\phantom{5' \quad CC}G\phantom{TC}G\phantom{TT}G\phantom{ACACCA}G$$

$$\phantom{5' \quad A^{AA}CACATCCA^ATT^T}A$$
$$5' \quad A^{AA}CACATCCA^ATT^TGC \quad 3'$$
$$\phantom{5' \quad A}GG\phantom{CACATCCA}G\phantom{TT}C$$
$$\phantom{5' \quad A^{AA}CACATCCA^ATT^T}G$$

**14.** Procédé de préparation de lysozyme humain, caractérisé par les étapes suivantes
  a. isolement d'un ARNm de lysozyme humain à partir de l'ARN total d'une lignée cellulaire humaine
  b. construction d'un ADNc à partir de l'isolat obtenu à l'étape a. puis clonage de l'ADNc double brin
  c. criblage de la banque d'ADNc obtenue à partir de l'étape b. par hybridation à l'aide d'oligonucléotides appropriés,
  d. construction de l'ADNc complet codant pour le lysozyme humain avec la séquence nucléotidique de formule suivante

5´AAGGTCTTTGAAAGGTGTGAGTTGGCCAGAACTCTGAAAAGATTGGGAATGGATGG
CTACAGGGGAATCAGCCTAGCAAACTGGATGTGTTTGGCCAAATGGGAGAGTGGTTAC
AACACACGAGCTACAAACTACAATGCTGGAGACAGAAGCACTGATTATGGGATATTTC
AGATCAATAGCCGCTACTGGTGTAATGATGGCAAAACCCCAGGAGCAGTTAATGCCTG
TCATTTATCCTGCAGTGCTTTGCTGCAAGATAACATCGCTGATGCTGTAGCTTGTGCA
AAGAGGGTTGTCCGTGATCCACAAGGCATTAGAGCATGGGTGGCATGGAGAAATCGTT
GTCAAAACAGAGATGTCCGTCAGTATGTTCAAGGTTGTGGAGTG

ou de ses variations alléliques avec la fonction de codage du lysozyme humain

ou d'une séquence nucléotidique qui s'hybride avec les formules des séquences nucléotidiques précédentes dans des conditions strictes qui opèrent une sélection pour plus de 85% d'homologie et qui code pour le lysozyme humain,

e. liaison d'un ADNc obtenu à partir de l'étape d. à son extrémité 5' avec une séquence de tête de levure,

f. recombinaison de l'ADN obtenu à partir de l'étape e. avec un ADN vectoriel et construction d'un plasmide d'expression qui est constitué essentiellement par les éléments promoteur, signal d'initiation de la traduction, une molécule d'ADN obtenue à partir de l'étape e., un codon d'arrêt et un terminateur,

g. transformation d'une cellule hôte appropriée avec une molécule d'ADN recombinée obtenue à partir de l'étape f., culture de ces cellules dans un milieu approprié et isolement à partir du milieu du lysozyme humain obtenu.

Fig.1

Fig. 2

ON 19

ON 20

dC.dG
tail

Leader
sequence

Coding   region

3'flanking
region

dC.dG
tail

CLONE 14-1

Fig. 3a

EcoRI
BamHI
Sal I

MnI I
Mae III

Bal I

Hinf I

Bal I
Mae III

Pst I

MnI I

Mae III

Hind III

Fig. 3b

100 bp

```
        10          20          30          40          50          60
CCCCCCCCCCCCCCCCCCCCATTGTTCTGGGGCTTGTCCTCCTTTCTGTTACGGTTCAAGG


        70          80          90         100         110         120
CAAGGTCTTTGAAAGGTGTGAGTTGGCCAGAACTCTGAAAAGATTGGGAATGGATGGCTA


       130         140         150         160         170         180
CAGGGGAATCAGCCTAGCAAACTGGATGTGTTTGGCCAAATGGGAGAGTGGTTACAACAC


       190         200         210         220         230         240
ACGAGCTACAAACTACAATGCTGGAGACAGAAGCACTGATTATGGGATATTTCAGATCAA


       250         260         270         280         290         300
TAGCCGCTACTGGTGTAATGATGGCAAAACCCCAGGAGCAGTTAATGCCTGTCATTTATC


       310         320         330         340         350         360
CTGCAGTGCTTTGCTGCAAGATAACATCGCTGATGCTGTAGCTTGTGCAAAGAGGGTTGT


       370         380         390         400         410         420
CCGTGATCCACAAGGCATTAGAGCATGGGTGGCATGGAGAAATCGTTGTCAAAACAGAGA


       430         440         450         460         470         480
TGTCCGTCAGTATGTTCAAGGTTGTGGAGTGTAACTCCAGAATTTTGGGGGGGGGGGGGG


       490
GGGGGGGGGG
```

# Fig. 4a

Fig. 4b

```
                                                        1
                                                LYS VAL PHE
ILE VAL LEU GLY LEU VAL LEU LEU SER VAL THR VAL GLN GLY LYS VAL PHE


                          10                                    20
GLU ARG CYS GLU LEU ALA ARG THR LEU LYS ARG LEU GLY MET ASP GLY TYR
GLU ARG CYS GLU LEU ALA ARG THR LEU LYS ARG LEU GLY MET ASP GLY TYR


                                        30
ARG GLY ILE SER LEU ALA ASN TRP MET CYS LEU ALA LYS TRP GLU SER GLY
ARG GLY ILE SER LEU ALA ASN TRP MET CYS LEU ALA LYS TRP GLU SER GLY


            40                                      50
TYR ASN THR ARG ALA THR ASN TYR ASN ALA GLY ASP ARG SER THR ASP TYR
TYR ASN THR ARG ALA THR ASN TYR ASN ALA GLY ASP ARG SER THR ASP TYR


                          60                                    70
GLY ILE PHE GLN ILE ASN SER ARG TYR TRP CYS ASN ASP GLY LYS THR PRO
GLY ILE PHE GLN ILE ASN SER ARG TYR TRP CYS ASN ASP GLY LYS THR PRO


                                        80
GLY ALA VAL ASN ALA CYS HIS LEU SER CYS SER ALA LEU LEU GLN ASP ASN
GLY ALA VAL ASN ALA CYS HIS LEU SER CYS SER ALA LEU LEU GLN ASP ASN


            90                                      100
ILE ALA ASP ALA VAL ALA CYS ALA LYS ARG VAL VAL ARG ASP PRO GLN GLY
ILE ALA ASP ALA VAL ALA CYS ALA LYS ARG VAL VAL ARG ASP PRO GLN GLY


                          110                                   120
ILE ARG ALA TRP VAL ALA TRP ARG ASN ARG CYS GLN ASN ARG ASP VAL ARG
ILE ARG ALA TRP VAL ALA TRP ARG ASN ARG CYS GLN ASN ARG ASP VAL ARG


                          130
GLN TYR VAL GLN GLY CYS GLY VAL
GLN TYR VAL GLN GLY CYS GLY VAL Ochre
```

# Fig. 5

Fig. 6

Fig. 7

Fig. 8

Fig. 9

Fig.10

Fig.11

Fig. 12

Fig. 13

Fig. 14a

C2 isoliert

pWS231D
PstI BglII

EcoRI    SalI    PstI                    BglII      EcoRI

α-F.-Promotor                                      pWS294D

3' HLZ

α-Faktor-
Terminator

PstI                    A2 mutagenisiert
                        PstI

SalI     PstI    PstI                   EcoRI

EcoRI

HLZ    α-Faktor-Promotor                            pWS296D

α-Faktor-
Terminator

Fig. 14 b